# EUROPEAN PATENT SPECIFICATION

(11) **EP 4 165 017 B1**
(45) Date of publication and mention of the grant of the patent: **04.09.2024**
(21) Application number: 21728953.7
(22) Date of filing: 07.06.2021
(51) Int. Cl.: C07C 251/60, C07C 251/58, C07D 317/46, C07D 213/65, A01N 35/10, A01N 37/36, A01N 37/50, A01N 43/30, A01N 43/40

(54) **USE OF STROBILURIN TYPE COMPOUNDS FOR COMBATING PHYTOPATHOGENIC FUNGI CONTAINING AN AMINO ACID SUBSTITUTION F129L IN THE MITOCHONDRIAL CYTOCHROME B PROTEIN CONFERRING RESISTANCE TO QO INHIBITORS V**
VERWENDUNG VON VERBINDUNGEN VOM STROBILURINTYP ZUR BEKÄMPFUNG VON PHYTOPATHOGENEN PILZEN, DIE EINE AMINOSÄURESUBSTITUTION F129L IN DEM MITOCHONDRIALEN CYTOCHROM-B-PROTEIN ENTHALTEN, DAS RESISTENZ GEGENÜBER QO-INHIBITOREN V VERLEIHT
UTILISATION DE COMPOSÉS DE TYPE STROBILURINE POUR LUTTER CONTRE LES CHAMPIGNONS PHYTOPATHOGÈNES CONTENANT UNE SUBSTITUTION D'ACIDE AMINÉ F129L DANS LA PROTÉINE MITOCHONDRIALE DU CYTOCHROME B CONFÉRANT UNE RÉSISTANCE AUX INHIBITEURS QO V

(30) Priority: 10.06.2020 IN 202021024406; 31.07.2020 EP 20188910; 31.05.2021 EP 21176864
(43) Date of publication of application: 19.04.2023
(73) Proprietor: BASF SE, 67056 Ludwigshafen am Rhein (DE)
(72) Inventor: KULKARNI, Sarang, Mumbai 400705 (IN); DEY, Chandan, Mumbai 400705 (IN); POONOTH, Manojkumar, 67056 Ludwigshafen (DE); LE VEZOUET, Ronan, 67056 Ludwigshafen (DE); LOHMANN, Jan Klaas, 67056 Ludwigshafen (DE); CRAIG, Ian Robert, 67056 Ludwigshafen (DE); GROTE, Thomas, 67157 Wachenheim (DE); KHANNA, Smriti, Mumbai 400705 (IN); RATH, Rakesh, Mumbai 400705 (IN); KOCH, Andreas, 67117 Limburgerhof (DE); FEHR, Marcus, 67117 Limburgerhof (DE); TEGGE, Vanessa, 67117 Limburgerhof (DE)
(74) Representative: BASF IP Association
(86) International application number: PCT/EP2021/065146
(87) International publication number: WO 2021/249928

(56) References cited:
- EP-A1- 0 463 488
- WO-A1-2017/157923
- WO-A1-2020/027216
- WO-A1-97/05103

## Description

The present invention relates the use of strobilurin type compounds of formula I and the N-oxides and the salts thereof for combating phytopathogenic fungi containing an amino acid substitution F129L in the mitochondrial cytochrome b protein (also referred to as F129L mutation in the mitochondrial cytochrome b gene) conferring resistance to Qo inhibitors (Qol), and to methods for combating such fungi. The invention also relates to novel compounds, processes for preparing these compounds, to compositions comprising at least one such compound, to plant health applications, and to seeds coated with at least one such compound. The present invention also relates to a method for controlling soybean rust fungi (*Phakopsora pachyrhizi*) with the amino acid substitution F129L in the mitochondrial cytochrome b protein.

"Qo inhibitor," as used herein, includes any substance that is capable of diminishing and/or inhibiting respiration by binding to a ubihydroquinone oxidation center of a cytochrome bc₁ complex in mitochondria. The oxidation center is typically located on the outer side of the inner mitochondrial membrane. Many of these compounds are also known as strobilurin-type or strobilurin analogue compounds.

The mutation F129L in the mitochondrial cytochrome b (CYTB) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the CYTB (cytochrome b) gene resulting in a single amino acid substitution in the position 129 from F to L in the cytochrome b protein. Such F129L mutation is known to confer resistance to Qo inhibitors.

Qol fungicides, often referred to as strobilurin-type fungicides, are conventionally used to control a number of fungal pathogens in crops. Qo inhibitors typically work by inhibiting respiration by binding to a ubihydroquinone oxidation center of a cytochrome bc₁ complex (electron transport complex III) in mitochondria. Said oxidation center is located on the outer side of the inner mitochondrial membrane. A prime example of the use of Qols includes the use of, for example, strobilurins on wheat for the control of *Septoria tritici* (also known as *Mycosphaerella graminicola*), which is the cause of wheat leaf blotch. Unfortunately, widespread use of such Qols has resulted in the selection of mutant pathogens which are resistant to such Qols. Resistance to Qols has been detected in several phytopathogenic fungi such as *Blumeria graminis, Mycosphaerella fijiensis, Pseudoperonspora cubensis* or *Venturia inaequalis.* The major part of resistance to Qols in agricultural uses has been attributed to pathogens containing a single amino acid residue substitution G143A in the cytochrome b gene for their cytochrome bc₁ complex, the target protein of Qols which have been found to be controlled by specific Qols (WO 2013/092224). Despite several commercial Qol fungicides have also been widely used in soybean rust control, the single amino acid residue substitution G143A in the cytochrome b protein conferring resistance to Qol fungicides was not observed.

Instead soybean rust acquired a different genetic mutation in the cytochrome b gene causing a single amino acid substitution F129L which also confers resistance against Qol fungicides. The efficacy of Qol fungicides used against soybean rust conventionally, i.e. pyraclostrobin, azoxystrobin, picoxystrobin, orysastrobin, dimoxystrobin and metominostrobin, has decreased to a level with practical problems for agricultural practice.

Although it seems that trifloxystrobin was less affected by the F129L amino acid substitution to the same degree as other Qol fungicides such as azoxystrobin and pyraclostrobin, trifloxystrobin was never as efficacious on a fungal population bearing the F129L Qol resistance mutation as on a sensitive population (Crop Protection 27, (2008) 427-435).

Thus, new methods are desirable for controlling pathogen induced diseases in crops comprising plants subjected to pathogens containing a F129L mutation in the mitochondrial cytochrome b gene conferring resistance to Qo inhibitors. Furthermore, in many cases, in particular at low application rates, the fungicidal activity of the known fungicidal strobilurin compounds is unsatisfactory, especially in case that a high proportion of the fungal pathogens contain a mutation in the mitochondrial cytochrome b gene conferring resistance to Qo inhibitors. Besides there is an ongoing need for new fungicidally active compounds which are more effective, less toxic and/or environmentally safer. Based on this, it was also an object of the present invention to provide compounds having improved activity and/or a broader activity spectrum against phytopathogenic fungi and/or even further reduced toxicity against non target organisms such as vertebrates and invertebrates.

Certain strobilurin type compounds have been described in WO 1997/05103 and EP 463488. However, it is not mentioned that these compounds inhibit fungal pathogens containing a F129L substitution in the mitochondrial cytochrome b protein conferring resistance to Qo inhibitors.

The compounds according to the present invention differ from those described in the abovementioned publications that the methyl oxime side chain contains an alkyne linker group as defined herein.

Therefore, the invention provides novel compounds of formula I 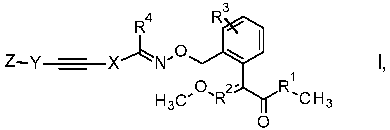 wherein
- R¹: is selected from O and NH;
- R²: is selected from CH and N;
- R³: is selected from hydrogen, halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
- R⁴: is selected from hydrogen, C₁-C₆-alkyl, C₂-C₄-alkenyl, C₂-C₄-alkynyl, C₁-C₆-haloalkyl, C₂-C₄-haloalkenyl, C₂-C₄-haloalkynyl, -(C₁-C₂-alkyl)-O-(C₁-C₂-alkyl), -(C₁-C₂-alkyl)-O-(C₁-C₂-haloalkyl) and -C₁-C₄-alkyl-C₃-C₆-cycloalkyl;
- X, Y ,: independently of each other, are a direct bond or the divalent group -CL¹L²-;
- L¹, L²,: independently of each other, are selected from hydrogen, C₁-C₃-alkyl, C₁-C₃-haloalkyl, C₂-C₃-haloalkenyl, C₂-C₃-haloalkynyl, -(C₁-C₂-alkyl)-Q-(C₁-C₂-alkyl), -(C₁-C₂-alkyl)-Q-(C₁-C₂-haloalkyl), cyclopropyl and -C₁-C₂-alkyl-cyclopropyl; or
L¹ and L², together with the interjacent carbon atom, form a cyclopropyl; wherein the cyclic moieties of L¹ and L², independently of each other, are unsubstituted or carry 1 or 2 identical or different groups R^{L}:
R^{L} is selected from halogen, CN, NO₂, C₁-C₄-alkyl, C₁-C₄-haloalkyl, -O-C₁-C₄-alkyl and -O-C₁-C₄-haloalkyl;
- Z: is selected from C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
and wherein Z is unsubstituted or carries 1, 2, 3 or up to the maximum number of identical or different groups R^{a}:

R^{a} is selected from halogen, CN, NR^{A}R^{B}, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -C(=O)-O-C₁-C₄-alkyl, -C(=O)-NH-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl, heterocycloalkenyl and heteroaryl are bound directly or via an oxygen atom or via a C₁-C₂-alkylene linker,
and/or

2 R^{a} substituents bound to neighboring carbon ring atoms, together with the two interjacent carbon ring atoms, form a partially unsaturated or aromatic 5- to 6-membered fused carbo- or heterocycle,
wherein the heterocycle includes beside carbon atoms 1 or 2 heteroatoms independently selected from N, O and S as ring member atoms, provided that such heterocycle cannot contain 2 contiguous atoms selected from O and S;
and wherein the aliphatic and cyclic moieties of R^{a} are unsubstituted or carry 1, 2, 3, 4 or up to the maximum number of identical or different groups R^{b}:
   - R^{b}: is selected from halogen, CN, NO₂, C₁-C₄-alkyl, C₁-C₄-haloalkyl, -O-C₁-C₄-alkyl, and -O-C₁-C₄-haloalkyl;
   - R^{A}, R^{B}: independently of each other, are selected from hydrogen, C₁-C₄-alkyl and C₁-C₄-haloalkyl;
   and in form or stereoisomers and tautomers thereof, and the N-oxides and the agriculturally acceptable salts thereof.

Although the present invention will be described with respect to particular embodiments, this description is not to be construed in a limiting sense.

Before describing in detail exemplary embodiments of the present invention, definitions important for understanding the present invention are given. As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of'.

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

The term "compounds I" refers to compounds of formula I. Likewise, this terminology applies to all sub-formulae, e. g. "compounds I.2" refers to compounds of formula I.2 or "compounds V" refers to compounds of formula V, etc..

The term "independently" when used in the context of selection of substituents for a variable, it means that where more than one substituent is selected from a number of possible substituents, those substituents may be the same or different.

The organic moieties or groups mentioned in the above definitions of the variables are collective terms for individual listings of the individual group members. The term "Cᵥ-C_{w}" indicates the number of carbon atom possible in each case.

The term "halogen" refers to fluorine, chlorine, bromine and iodine.

The term "C₁-C₄-alkyl" refers to a straight-chained or branched saturated hydrocarbon group having 1 to 4 carbon atoms, for example, methyl (CH₃), ethyl (C₂H₅), propyl, 1-methylethyl (isopropyl), butyl, 1-methylpropyl, 2-methylpropyl, 1,1-dimethylethyl.

The term "C₂-C₄-alkenyl" refers to a straight-chain or branched unsaturated hydrocarbon radical having 2 to 4 carbon atoms and a double bond in any position such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₄-alkynyl" refers to a straight-chain or branched unsaturated hydrocarbon radical having 2 to 4 carbon atoms and containing at least one triple bond such as ethynyl, prop-1-ynyl, prop-2-ynyl, but-1-ynyl, but-2-ynyl, but-3-ynyl, 1-methyl-prop-2-ynyl.

The term "C₁-C₄-haloalkyl" refers to a straight-chained or branched alkyl group having 1 to 4 carbon atoms wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "-O-C₁-C₄-alkyl" refers to a straight-chain or branched alkyl group having 1 to 4 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methyl¬propoxy, 2-methylpropoxy or 1,1-dimethylethoxy.

The term "C₃-C₆-cycloalkyl" refers to monocyclic saturated hydrocarbon radicals having 3 to 6 carbon ring members, such as cyclopropyl (C₃H₅), cyclobutyl, cyclopentyl or cyclohexyl. The term "C₃-C₆-cycloalkenyl " refers to monocyclic saturated hydrocarbon radicals having 3 to 6 carbon ring members and one or more double bonds.

The term "3- to 6-membered heterocycloalkyl" refers to 3- to 6-membered monocyclic saturated ring system having besides carbon atoms one or more heteroatoms, such as O, N, S as ring members. The term "C₃-C₆-membered heterocycloalkenyl" refers to 3- to 6-membered monocyclic ring system having besides carbon atoms one or more heteroatoms, such as O, N and S as ring members, and one or more double bonds.

The term "-C₁-C₄-alkyl-C₃-C₆-cycloalkyl" refers to alkyl having 1 to 4 carbon atoms (as defined above), wherein one hydrogen atom of the alkyl radical is replaced by a cycloalkyl radical having 3 to 6 carbon atoms.

The term "phenyl" refers to C₆H₅.

The term "5- or 6-membered heteroaryl" which contains 1, 2, 3 or 4 heteroatoms from the group consisting of O, N and S, is to be understood as meaning aromatic heterocycles having 5 or 6 ring atoms. Examples include:
- 5-membered heteroaryl which in addition to carbon atoms, e.g. contain 1, 2 or 3 N atoms and/or one sulfur and/or one oxygen atom: for example 2-thienyl, 3-thienyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl and 1,3,4-triazol-2-yl;
- 6-membered heteroaryl which, in addition to carbon atoms, e.g. contain 1, 2, 3 or 4 N atoms as ring members, e.g. 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

The term "C₁-C₂-alkylene linker" means a divalent alkyl group such as -CH₂- or -CH₂-CH₂-that is bound at one end to the core structure of formula I and at the other end to the particular substituent.

As used herein, the "compounds", in particular "compounds I" include all the stereoisomeric and tautomeric forms and mixtures thereof in all ratios, isotopic forms, their agriculturally acceptable salts and N-oxides thereof.

The term "stereoisomer" is a general term used for all isomers of individual compounds that differ only in the orientation of their atoms in space. The term stereoisomer includes mirror image isomers (enantiomers), mixtures of mirror image isomers (racemates, racemic mixtures), geometric (cis/trans or E/Z) isomers, and isomers of compounds with more than one chiral center that are not mirror images of one another (diastereoisomers). The term "tautomer" refers to the coexistence of two (or more) compounds that differ from each other only in the position of one (or more) mobile atoms and in electron distribution, for example, keto-enol tautomers. The term "agriculturally acceptable salts" as used herein, includes salts of the active compounds which are prepared with acids or bases, depending on the particular substituents found on the compounds described herein. "N-oxide" refers to the oxide of the nitrogen atom of a nitrogen-containing heteroaryl or heterocycle. N-oxide can be formed in the presence of an oxidizing agent for example peroxide such as m-chloro-perbenzoic acid or hydrogen peroxide. N-oxide refers to an amine oxide, also known as amine-N-oxide, and is a chemical compound that contains N→O bond.

In respect of the variables, the embodiments of the intermediates correspond to the embodiments of the compounds I.

Preference is given to those compounds I and where applicable also to compounds of all sub-formulae provided herein, e. g. formulae !.1 and I.2, and to the intermediates such as compounds II, III, IV and V, wherein the substituents and variables (such as n, X, Y, Z, R¹, R², R³, R⁴, R^{a}, and R^{b}) have independently of each other or more preferably in combination (any possible combination of 2 or more substituents as defined herein) the following meanings:

Preference is also given to the uses, methods, mixtures and compositions, wherein the definitions (such as phytopathogenic fungi, treatments, crops, compounds II, further active ingredients, solvents, solid carriers) have independently of each other or more preferably in combination the following meanings and even more preferably in combination (any possible combination of 2 or more definitions as provided herein) with the preferred meanings of compounds I herein:
One embodiment of the invention relates to preferred compounds I, wherein R¹ is selected from O and NH; and R² is selected from CH and N, provided that R² is N in case R¹ is NH. Another embodiment related to compounds wherein R² is N. A further embodiment relates to compounds I, wherein R² is CH.

According to a further embodiment, R³ is selected from hydrogen, halogen, CN, C₁-C₄-alkyl, C₂-C₄-alkenyl, C₁-C₄-haloalkyl, C₂-C₄-haloalkenyl, C₃-C₆-cycloalkyl, -O-C₁-C₄-alkyl, -O-C₁-C₄-haloalkyl, -C₁-C₂-alkyl-C₃-C₆-cycloalkyl and 3- to 6-membered heterocycloalkyl; more preferably from is selected from hydrogen, halogen, CN, C₁-C₄-alkyl, C₂-C₄-alkenyl, C₂-C₄-alkynyl, C₁-C₄-haloalkyl, C₃-C₄-cycloalkyl, -O-C₁-C₄-alkyl, -O-C₁-C₄-haloalkyl and 3- to 4-membered heterocycloalkyl, wherein said heterocycloalkyl besides carbon atoms contain 1 or 2 heteroatoms selected from N, O and S provided that such heterocycle cannot contain 2 contiguous atoms selected from O and S, and wherein said heterocycloalkyl is bound directly or via an oxygen atom or via a C₁-C₂-alkylene linker; even more preferably from hydrogen, C₁-C₂-alkyl, C₂-alkenyl, C₁-C₂-haloalkyl, -O-C₁-C₂-alkyl, O-C₁-C₂-haloalkyl, C₃-C₄-cycloalkyl, -C₁-C₂-alkyl-C₃-C₄-cycloalkyl, and 3- to 4-membered heterocycloalkyl; further more preferably form hydrogen, C₁-C₂-alkyl, C₁-C₂-haloalkyl, C₃-C₄-cycloalkyl, -O-C₁-C₂-alkyl and -O-C₁-C₂-haloalkyl; particularly preferred from hydrogen, halogen, C₁-C₂-alkyl, and C₁-C₂-haloalkyl, in particular hydrogen or methyl.

Preferably, R³ is in ortho position to the methyl oxime side chain of the molecule, which compounds are of formula I.A:

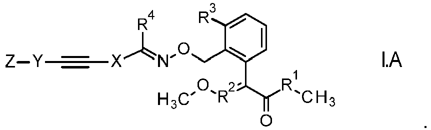

According to a further embodiment, R⁴ is selected from is selected from hydrogen, C₁-C₄-alkyl, C₂-C₄-alkenyl, C₁-C₄-haloalkyl, C₂-C₄-haloalkenyl, -(C₁-C₂-alkyl)-O-(C₁-C₂-alkyl) and -CH₂-cyclopropyl; more preferably from hydrogen, C₁-C₄-alkyl and C₁-C₄-haloalkyl, even more preferably from methyl and C₁-haloalkyl; in particular methyl.

According to a further embodiment, X is a direct bond.

According to a further embodiment, Y is a direct bond. More preferably, X and Y are direct bonds.

According to a further embodiment, at least one of X and Y is a divalent group -CL¹L²-.

According to a further embodiment, L¹ and L², independently of each other, are selected from hydrogen, C₁-C₃-alkyl, C₁-C₃-haloalkyl, cyclopropyl and -CH₂-cyclopropyl; or, L¹ and L², together with the interjacent carbon atom, form a cyclopropyl; more preferably L¹ and L², independently of each other, are selected from hydrogen and C₁-C₃-alkyl; or, L¹ and L², together with the interjacent carbon atom, form a cyclopropyl; in particular L¹ and L² are both hydrogen.

According to a further embodiment, Z is selected from C₃-C₆-cycloalkyl, phenyl and 5- or 6-membered heteroaryl, wherein said heteroaryl besides carbon atoms contain 1, 2 or 3 heteroatoms selected from N, O and S provided that such heteroaryl cannot contain 2 contiguous atoms selected from O and S, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to a further embodiment, Z is selected from C₃-C₆-cycloalkyl, phenyl and 5- or 6-membered heteroaryl, wherein said heteroaryl besides carbon atoms contain 1, 2 or 3 heteroatoms selected from N, O and S provided that such heteroaryl cannot contain 2 contiguous atoms selected from O and S, and wherein Z carries 1, 2 or 3 identical or different groups R^{a} as defined herein; more preferably Z carries 1 or 2 identical or different groups R^{a} as defined herein; in particular Z carries 1 group R^{a} as defined herein.

According to a further embodiment, Z is selected from cyclopropyl and phenyl, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to a further embodiment, Z is phenyl and wherein Z is unsubstituted or carries 1, 2 or 3 identical or different groups R^{a} as defined herein and/or 2 R^{a} substituents bound to neighboring carbon ring atoms, together with the two interjacent carbon ring atoms, form a partially unsaturated or aromatic 5- to 6-membered fused carbo- or heterocycle, wherein the heterocycle includes beside carbon atoms 1 or 2 heteroatoms independently selected from N, O and S as ring member atoms, provided that such heterocycle cannot contain 2 contiguous atoms selected from O and S; and wherein the R^{a} are unsubstituted or carry 1, 2, 3, 4 or up to the maximum number of identical or different groups R^{b} being selected from halogen, CN, NO₂, C₁-C₄-alkyl, C₁-C₄-haloalkyl, -O-C₁-C₄-alkyl, and -O-C₁-C₄-haloalkyl. Said fused carbocycle may be a cyclopentene ring (resulting together with the fused phenyl in an indane bicyclic ring system), a cyclopentadiene ring (resulting together with the fused phenyl in an 1H-indene bicyclic ring system), a cyclohexene ring (resulting together with the condensed phenyl ring in a tetralin bicyclic ring system), a cyclohexadiene ring (resulting together with the fused phenyl in a dihydronaphthalene bicyclic ring system), a cycloheptene ring (resulting together with the fused phenyl in a 6,7,8,9-tetrahydro-5H-benzo[7]annulene bicyclic ring system). Said fused heterocycle may be a 2,3-dihydrofuran ring (resulting together with the fused phenyl in an 2,3-dihydrobenzofuran bicyclic ring system), a 3,4-dihydro-2H-pyran ring (resulting together with the fused phenyl in a chromane bicyclic ring system), a furan ring (resulting together with the fused phenyl ring in a benzofuran bicyclic ring system), a 1,3-dioxole ring (resulting together with the fused phenyl ring in a benzo-1,3-dioxole bicyclic ring system), a 2,3-dihydro-1H-pyrrole ring (resulting together with the fused phenyl ring in a indoline bicyclic ring system), a 1,3-dihydro-pyrrol-2-one ring (resulting together with the fused phenyl ring in a indolin-2-one bicyclic ring system), more preferably said fused heterocycle my be a 1,3-dioxole ring (resulting together with the fused phenyl ring in a benzo-1,3-dioxole bicyclic ring system) that is bound to the side chain in position 5 resulting in Z, together with two R^{a} substituents, forming a 1,3-benzodioxol-5-yl.

According to the abovementioned embodiments, R^{a} is preferably selected from halogen, CN, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₄-cycloalkyl, -C₁-C₂-alkyl-C₃-C₄-cycloalkyl, -O-C₃-C₄-cycloalkyl, phenyl, 3- to 5-membered heterocycloalkyl, 3- to 5-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, wherein said phenyl, heterocycloalkyl, heterocycloalkenyl and heteroaryl are bound directly or via an oxygen atom or via a C₁-C₂-alkylene linker.

Preferably, R^{a} is selected from halogen, CN, C₁-C₄-alkyl, C₂-C₄-alkenyl, C₂-C₄-alkynyl, -O-C₁-C₄-alkyl, -C(=O)-C₁-C₂-alkyl, -C(=N-O-C₁-C₄-alkyl)-C₁-C₄-alkyl, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker.

More preferably, R^{a} is selected from halogen, CN, C₁-C₃-alkyl, -O-C₁-C₃-alkyl, -C(=N-O-CH₃)-CH₃, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker.

In particular, R^{a} is selected from halogen, CN, C₁-C₂-alkyl, -O-C₁-C₂-alkyl, ethenyl, ethynyl and -C(=N-O-CH₃)-CH₃.

According to a further embodiment, R^{a} is selected from halogen, C₁-C₂-alkyl, -O-C₁-C₂-alkyl, wherein the aliphatic moieties are unsubstituted or carry 1, 2 or 3 identical or different groups R^{b} selected from halogen.

According to the abovementioned embodiments for R^{a}, the abovementioned heterocycloalkyl is more preferably a 4-membered heterocycloalkyl, wherein said heterocycloalkyl besides carbon atoms contains 1 heteroatom selected from N, O and S, preferably N.

According to the abovementioned embodiments for R^{a}, the abovementioned heteroaryl is more preferably a 5-membered heteroaryl, wherein said heteroaryl besides carbon atoms contains 1 or 2 heteroatoms selected from N, O and S provided that such heteroaryl cannot contain 2 contiguous atoms selected from O and S, preferably the heteroatoms are selected from N and O.

According to the abovementioned embodiments for R^{a}, the aliphatic and cyclic moieties of R^{a} are unsubstituted or carry 1, 2, 3, 4 or up to the maximum number of identical or different groups R^{b} selected from halogen, CN, NO₂, C₁-C₄-alkyl, C₁-C₄-haloalkyl, -O-C₁-C₄-alkyl and -O-C₁-C₄-haloalkyl; more preferably from halogen; even more preferably only the cyclic moieties of R^{a} are unsubstituted or carry 1, 2, 3, 4 or up to the maximum number of identical or different groups R^{b} selected from halogen, CN, NO₂, C₁-C₄-alkyl, C₁-C₄-haloalkyl, -O-C₁-C₄-alkyl and -O-C₁-C₄-haloalkyl; even more preferably only the phenyl moiety of R^{a} is unsubstituted or carries 1, 2, 3, 4 or 5 identical or different groups R^{b} selected from halogen, CN, C₁-C₄-alkyl, C₁-C₄-haloalkyl, -O-C₁-C₄-alkyl and -O-C₁-C₄-haloalkyl; in particular said phenyl is unsubstituted or carries 1, 2 or 3 identical or different groups R^{b} selected from halogen, CN, C₁-C₂-alkyl, C₁-C₂-haloalkyl, -O-C₁-C₂-alkyl and -O-C₁-C₂-haloalkyl.

According to a further embodiment, X and Y are both direct bonds, and Z is phenyl which is unsubstituted or carries 1, 2 or 3 identical or different substituents R^{a}, wherein R^{a} is selected from halogen, C₁-C₂-alkyl and -O-C₁-C₂-alkyl, wherein the aliphatic moieties of R^{a} are unsubstituted or carry 1, 2 or 3 identical or different groups R^{b} selected from halogen.

According to a further preferred embodiment, the present invention relates to compounds of formula I wherein:
- R¹: is selected from O and NH;
- R²: is CH or N;
- R³: is hydrogen, halogen or C₁-C₄-alkyl, wherein R³ is in ortho position to the methyl oxime side chain;
- R⁴: is selected from C₁-C₆-alkyl;
- X, Y: are both direct bonds;
- Z: is selected from cyclopropyl, phenyl and 5-membered heteroaryl, wherein said heteroaryl besides carbon atoms contains 1, 2 or 3 heteroatoms selected from N, O and S provided that such heteroaryl cannot contain 2 contiguous atoms selected from O and S, and wherein Z is unsubstituted or carries 1, 2, 3 or up to the maximum number of identical or different groups R^{a}:
R^{a} is selected from halogen, CN, C₁-C₄-haloalkyl, C₁-C₄-alkyl, -O-C₁-C₄-alkyl, -O-C₁-C₄-haloalkyl, -C(=N-O-C₁-C₄-alkyl)-C₁-C₄-alkyl, -C(=O)-C₁-C₄-alkyl, C₃-C₄-cycloalkyl, -C₁-C₂-alkyl-C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker, and wherein the abovementioned cyclic moieties of R^{a} are unsubstituted or carry 1, 2 or 3 identical or different groups R^{b} selected from halogen, CN, C₁-C₂-alkyl, C₁-C₂-haloalkyl, -O-C₁-C₂-alkyl and -O-C₁-C₂-haloalkyl;
and/or
2 R^{a} substituents bound to neighboring carbon ring atoms, together with the two interjacent carbon ring atoms, form a partially unsaturated or aromatic 5- to 6-membered fused carbo- or heterocycle,
wherein the heterocycle includes beside carbon atoms 1 or 2 heteroatoms independently selected from N, O and S as ring member atoms, provided that such heterocycle cannot contain 2 contiguous atoms selected from O and S;
and in form or stereoisomers and tautomers thereof, and the N-oxides and the agriculturally acceptable salts thereof.

According to a further embodiment, R³ is in ortho position to the methyl oxime side chain, R¹ is NH and R² is N, which compounds are of formula I.A1:

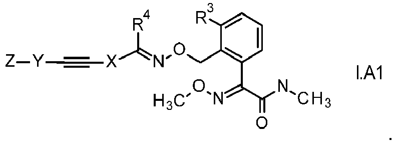

According to a further embodiment, R³ is in ortho position to the methyl oxime side chain, R¹ is O and R² is N, which compounds are of formula I.A2:

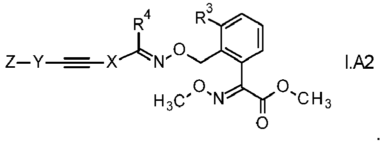

According to a further embodiment, R³ is in ortho position to the methyl oxime side chain, R¹ is O and R² is CH, which compounds are of formula I.A3:

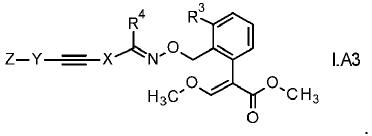

According to a further embodiment, R¹ is NH, R² is N and R³ is H, which compounds are of formula I.B1:

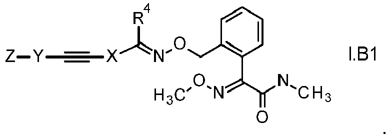

According to a further embodiment, R¹ is O, R² is N and R³ is H, which compounds are of formula I.B2:

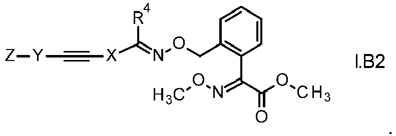

According to a further embodiment, R¹ is O, R² is CH and R³ is H, which compounds are of formula I.B3:

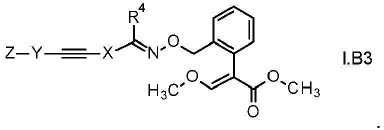

Preferably, R³ of compounds I is one of the following radicals 3-1 to 3-8:

| **No.** | **R³** |
|---|---|
| 3-1 | CH₃ |
| 3-2 | OCH₃ |
| 3-3 | CHF₂ |
| 3-4 | C₃H₅ |
| 3-5 | CH=CH₂ |
| 3-6 | CH₂CH=C(CH₃)₂ |
| 3-7 | CF₃ |
| 3-8 | C(=NOCH₃)CH₃ |
| 3-9 | H |

Even more preferably R³ is H, CH₃, OCH₃, CF₃, CHF₂ or C₃H₅, in particular H or CH₃.

Particularly preferred embodiments of the invention relate to compounds I, wherein the R⁴ is one of the following radicals 4-1 to 4-10:

| **No.** | **R⁴** |
|---|---|
| 4-1 | CH₃ |
| 4-2 | C₂H₅ |
| 4-3 | CH₂OCH₃ |
| 4-4 | CH₂CF₃ |
| 4-5 | CH₂C₆H₅ |
| 4-6 | CHF₂ |
| 4-7 | CH₂C₃H₅ |
| 4-8 | CH₂-C(=NOCH₃)CH₃ |
| 4-9 | C≡CH |
| 4-10 | C≡CCH₃ |

Particularly preferred embodiments of the invention relate to compounds I, wherein X and Y are direct bonds and Z is phenyl. Even more preferably, X and Y are direct bonds, Z is phenyl, R³ is in ortho position to the methyl oxime side chain, R¹ is NH and R² is N, wherein the phenyl Z is unsubstituted or substituted by 1, 2 or 3 groups R^{a} as defined herein, which compounds are of formula I.A1.1:

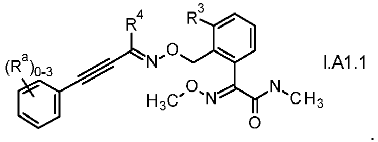

According to a further embodiment, X and Y are direct bonds, Z is phenyl, R³ is in ortho position to the methyl oxime side chain, R¹ is O and R² is N, wherein the phenyl Z is unsubstituted or substituted by 1, 2 or 3 groups R^{a} as defined herein, which compounds are of formula I.A2.1:

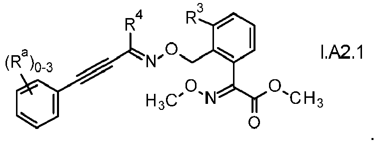

According to a further embodiment, X and Y are direct bonds, Z is phenyl, R³ is in ortho position to the methyl oxime side chain, R¹ is O and R² is CH, wherein the phenyl Z is unsubstituted or substituted by 1, 2 or 3 groups R^{a} as defined herein, which compounds are of formula I.A3.1:

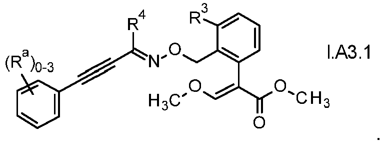

According to a further embodiment, X and Y are direct bonds, Z is phenyl, R¹ is NH, R² is N and R³ is H, wherein the phenyl Z is unsubstituted or substituted by 1, 2 or 3 groups R^{a} as defined herein, which compounds are of formula I.B1.1:

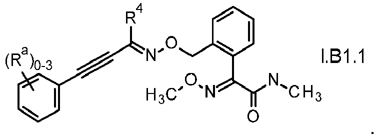

According to a further embodiment, X and Y are direct bonds, Z is phenyl, R¹ is O, R² is N and R³ is H, wherein the phenyl Z is unsubstituted or substituted by 1, 2 or 3 groups R^{a} as defined herein, which compounds are of formula I.B2.1:

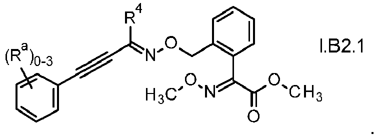

According to a further embodiment, X and Y are direct bonds, Z is phenyl, R¹ is O, R² is CH and R³ is H, wherein the phenyl Z is unsubstituted or substituted by 1, 2 or 3 groups R^{a} as defined herein, which compounds are of formula I.B3.1:

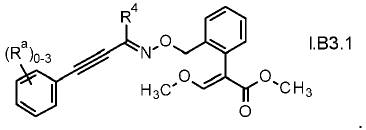

Particularly preferred embodiments of the invention relate to compounds I, wherein the R^{a} is selected of one of the following radicals a-1 to a-17:

| **No.** | **R^{a}** |
|---|---|
| a-1 | F |
| a-2 | Cl |
| a-3 | Br |
| a-4 | CH₃ |
| a-5 | CHF₂ |
| a-6 | CF₃ |
| a-7 | OCH₃ |
| a-8 | OCHF₂ |
| a-9 | OCF₃ |
| a-10 | C₂H₅ |
| a-11 | CH₂CF₃ |
| a-12 | CH=CH₂ |
| a-13 | C₆H₅ |
| a-14 | C≡CH |
| a-15 | C≡CCH₃ |
| a-16 | C₃H₅ |
| a-17 | C(=NOCH₃)CH₃ |

In an embodiment, compounds I are of formula I.A1.1 wherein R³ is CH₃ and R^{a} and R⁴ are as per any row of Table A below, which compounds are named I.A1.1-A-1 to I.A1.1-A-552.

In another embodiment, compounds I are of formula I.A2.1 wherein R³ is CH₃ and R^{a} and R⁴ are as per any row of Table A below, which compounds are named I.A2.1-A-1 to I.A2.1-A-552.

In another embodiment, compounds I are of formula I.A3.1 wherein R³ is CH₃ and R^{a} and R⁴ are as per any row of Table A below, which compounds are named I.A3.1-A-1 to I.A3.1-A-552.

In another embodiment, compounds I are of formula I.B1.1 and R^{a} and R⁴ are as per any row of Table A below, which compounds are named I.B1.1-A-1 to I.B1.1-A-552.

In another embodiment, compounds I are of formula I.B2.1 and R^{a} and R⁴ are as per any row of Table A below, which compounds are named I.B2.1-A-1 to I.B2.1-A-552.

In another embodiment, compounds I are of formula I.B3.1 and R^{a} and R⁴ are as per any row of Table A below, which compounds are named I.B3.1-A-1 to I.B3.1-A-552.

**Table A:**

| **No.** | **(R^{a})₀₋₃** | **R⁴** |
|---|---|---|
| A-1 | - | CH₃ |
| A-2 | 2-F | CH₃ |
| A-3 | 2-Cl | CH₃ |
| A-4 | 2-Br | CH₃ |
| A-5 | 2-CH₃ | CH₃ |
| A-6 | 2-CHF₂ | CH₃ |
| A-7 | 2-CF₃ | CH₃ |
| A-8 | 2-OCH₃ | CH₃ |
| A-9 | 2-OCHF₂ | CH₃ |
| A-10 | 2-OCF₃ | CH₃ |
| A-11 | 2-C₂H₅ | CH₃ |
| A-12 | 2-CH₂CF₃ | CH₃ |
| A-13 | 2-CH=CH₂ | CH₃ |
| A-14 | 2-C₆H₅ | CH₃ |
| A-15 | 2-C≡CH | CH₃ |
| A-16 | 2-C≡CCH₃ | CH₃ |
| A-17 | 2-C₃H₅ | CH₃ |
| A-18 | 2-C(=NOCH₃)CH₃ | CH₃ |
| A-19 | 2-CN | CH₃ |
| A-20 | 3-F | CH₃ |
| A-21 | 3-Cl | CH₃ |
| A-22 | 3-Br | CH₃ |
| A-23 | 3-CH₃ | CH₃ |
| A-24 | 3-CHF₂ | CH₃ |
| A-25 | 3-CF₃ | CH₃ |
| A-26 | 3-OCH₃ | CH₃ |
| A-27 | 3-OCHF₂ | CH₃ |
| A-28 | 3-OCF₃ | CH₃ |
| A-29 | 3-C₂H₅ | CH₃ |
| A-30 | 3-CH₂CF₃ | CH₃ |
| A-31 | 3-CH=CH₂ | CH₃ |
| A-32 | 3-C₆H₅ | CH₃ |
| A-33 | 3-C≡CH | CH₃ |
| A-34 | 3-C≡CCH₃ | CH₃ |
| A-35 | 3-C₃H₅ | CH₃ |
| A-36 | 3-C(=NOCH₃)CH₃ | CH₃ |
| A-37 | 3-CN | CH₃ |
| A-38 | 4-F | CH₃ |
| A-39 | 4-Cl | CH₃ |
| A-40 | 4-Br | CH₃ |
| A-41 | 4-CH₃ | CH₃ |
| A-42 | 4-CHF₂ | CH₃ |
| A-43 | 4-CF₃ | CH₃ |
| A-44 | 4-OCH₃ | CH₃ |
| A-45 | 4-OCHF₂ | CH₃ |
| A-46 | 4-OCF₃ | CH₃ |
| A-47 | 4-C₂H₅ | CH₃ |
| A-48 | 4-CH₂CF₃ | CH₃ |
| A-49 | 4-CH=CH₂ | CH₃ |
| A-50 | 4-C₆H₅ | CH₃ |
| A-51 | 4-C≡CH | CH₃ |
| A-52 | 4-C≡CCH₃ | CH₃ |
| A-53 | 4-C₃H₅ | CH₃ |
| A-54 | 4-C(=NOCH₃)CH₃ | CH₃ |
| A-55 | 4-CN | CH₃ |
| A-56 | - | C₂H₅ |
| A-57 | 2-F | C₂H₅ |
| A-58 | 2-Cl | C₂H₅ |
| A-59 | 2-Br | C₂H₅ |
| A-60 | 2-CH₃ | C₂H₅ |
| A-61 | 2-CHF₂ | C₂H₅ |
| A-62 | 2-CF₃ | C₂H₅ |
| A-63 | 2-OCH₃ | C₂H₅ |
| A-64 | 2-OCHF₂ | C₂H₅ |
| A-65 | 2-OCF₃ | C₂H₅ |
| A-66 | 2-C₂H₅ | C₂H₅ |
| A-67 | 2-CH₂CF₃ | C₂H₅ |
| A-68 | 2-CH=CH₂ | C₂H₅ |
| A-69 | 2-C₆H₅ | C₂H₅ |
| A-70 | 2-C≡CH | C₂H₅ |
| A-71 | 2-C≡CCH₃ | C₂H₅ |
| A-72 | 2-C₃H₅ | C₂H₅ |
| A-73 | 2-C(=NOCH₃)CH₃ | C₂H₅ |
| A-74 | 2-CN | C₂H₅ |
| A-75 | 3-F | C₂H₅ |
| A-76 | 3-Cl | C₂H₅ |
| A-77 | 3-Br | C₂H₅ |
| A-78 | 3-CH₃ | C₂H₅ |
| A-79 | 3-CHF₂ | C₂H₅ |
| A-80 | 3-CF₃ | C₂H₅ |
| A-81 | 3-OCH₃ | C₂H₅ |
| A-82 | 3-OCHF₂ | C₂H₅ |
| A-83 | 3-OCF₃ | C₂H₅ |
| A-84 | 3-C₂H₅ | C₂H₅ |
| A-85 | 3-CH₂CF₃ | C₂H₅ |
| A-86 | 3-CH=CH₂ | C₂H₅ |
| A-87 | 3-C₆H₅ | C₂H₅ |
| A-88 | 3-C≡CH | C₂H₅ |
| A-89 | 3-C≡CCH₃ | C₂H₅ |
| A-90 | 3-C₃H₅ | C₂H₅ |
| A-91 | 3-C(=NOCH₃)CH₃ | C₂H₅ |
| A-92 | 3-CN | C₂H₅ |
| A-93 | 4-F | C₂H₅ |
| A-94 | 4-Cl | C₂H₅ |
| A-95 | 4-Br | C₂H₅ |
| A-96 | 4-CH₃ | C₂H₅ |
| A-97 | 4-CHF₂ | C₂H₅ |
| A-98 | 4-CF₃ | C₂H₅ |
| A-99 | 4-OCH₃ | C₂H₅ |
| A-100 | 4-OCHF₂ | C₂H₅ |
| A-101 | 4-OCF₃ | C₂H₅ |
| A-102 | 4-C₂H₅ | C₂H₅ |
| A-103 | 4-CH₂CF₃ | C₂H₅ |
| A-104 | 4-CH=CH₂ | C₂H₅ |
| A-105 | 4-C₆H₅ | C₂H₅ |
| A-106 | 4-C≡CH | C₂H₅ |
| A-107 | 4-C≡CCH₃ | C₂H₅ |
| A-108 | 4-C₃H₅ | C₂H₅ |
| A-109 | 4-C(=NOCH₃)CH₃ | C₂H₅ |
| A-110 | 4-CN | C₂H₅ |
| A-111 | - | CH₂CF₃ |
| A-112 | 2-F | CH₂CF₃ |
| A-113 | 2-Cl | CH₂CF₃ |
| A-114 | 2-Br | CH₂CF₃ |
| A-115 | 2-CH₃ | CH₂CF₃ |
| A-116 | 2-CHF₂ | CH₂CF₃ |
| A-117 | 2-CF₃ | CH₂CF₃ |
| A-118 | 2-OCH₃ | CH₂CF₃ |
| A-119 | 2-OCHF₂ | CH₂CF₃ |
| A-120 | 2-OCF₃ | CH₂CF₃ |
| A-121 | 2-C₂H₅ | CH₂CF₃ |
| A-122 | 2-CH₂CF₃ | CH₂CF₃ |
| A-123 | 2-CH=CH₂ | CH₂CF₃ |
| A-124 | 2-C₆H₅ | CH₂CF₃ |
| A-125 | 2-C≡CH | CH₂CF₃ |
| A-126 | 2-C≡CCH₃ | CH₂CF₃ |
| A-127 | 2-C₃H₅ | CH₂CF₃ |
| A-128 | 2-C(=NOCH₃)CH₃ | CH₂CF₃ |
| A-129 | 2-CN | CH₂CF₃ |
| A-130 | 3-F | CH₂CF₃ |
| A-131 | 3-Cl | CH₂CF₃ |
| A-132 | 3-Br | CH₂CF₃ |
| A-133 | 3-CH₃ | CH₂CF₃ |
| A-134 | 3-CHF₂ | CH₂CF₃ |
| A-135 | 3-CF₃ | CH₂CF₃ |
| A-136 | 3-OCH₃ | CH₂CF₃ |
| A-137 | 3-OCHF₂ | CH₂CF₃ |
| A-138 | 3-OCF₃ | CH₂CF₃ |
| A-139 | 3-C₂H₅ | CH₂CF₃ |
| A-140 | 3-CH₂CF₃ | CH₂CF₃ |
| A-141 | 3-CH=CH₂ | CH₂CF₃ |
| A-142 | 3-C₆H₅ | CH₂CF₃ |
| A-143 | 3-C≡CH | CH₂CF₃ |
| A-144 | 3-C≡CCH₃ | CH₂CF₃ |
| A-145 | 3-C₃H₅ | CH₂CF₃ |
| A-146 | 3-C(=NOCH₃)CH₃ | CH₂CF₃ |
| A-147 | 3-CN | CH₂CF₃ |
| A-148 | 4-F | CH₂CF₃ |
| A-149 | 4-Cl | CH₂CF₃ |
| A-150 | 4-Br | CH₂CF₃ |
| A-151 | 4-CH₃ | CH₂CF₃ |
| A-152 | 4-CHF₂ | CH₂CF₃ |
| A-153 | 4-CF₃ | CH₂CF₃ |
| A-154 | 4-OCH₃ | CH₂CF₃ |
| A-155 | 4-OCHF₂ | CH₂CF₃ |
| A-156 | 4-OCF₃ | CH₂CF₃ |
| A-157 | 4-C₂H₅ | CH₂CF₃ |
| A-158 | 4-CH₂CF₃ | CH₂CF₃ |
| A-159 | 4-CH=CH₂ | CH₂CF₃ |
| A-160 | 4-C₆H₅ | CH₂CF₃ |
| A-161 | 4-C≡CH | CH₂CF₃ |
| A-162 | 4-C≡CCH₃ | CH₂CF₃ |
| A-163 | 4-C₃H₅ | CH₂CF₃ |
| A-164 | 4-C(=NOCH₃)CH₃ | CH₂CF₃ |
| A-165 | 4-CN | CH₂CF₃ |
| A-166 | - | CH₂OCH₃ |
| A-167 | 2-F | CH₂OCH₃ |
| A-168 | 2-Cl | CH₂OCH₃ |
| A-169 | 2-Br | CH₂OCH₃ |
| A-170 | 2-CH₃ | CH₂OCH₃ |
| A-171 | 2-CHF₂ | CH₂OCH₃ |
| A-172 | 2-CF₃ | CH₂OCH₃ |
| A-173 | 2-OCH₃ | CH₂OCH₃ |
| A-174 | 2-OCHF₂ | CH₂OCH₃ |
| A-175 | 2-OCF₃ | CH₂OCH₃ |
| A-176 | 2-C₂H₅ | CH₂OCH₃ |
| A-177 | 2-CH₂CF₃ | CH₂OCH₃ |
| A-178 | 2-CH=CH₂ | CH₂OCH₃ |
| A-179 | 2-C₆H₅ | CH₂OCH₃ |
| A-180 | 2-C≡CH | CH₂OCH₃ |
| A-181 | 2-C≡CCH₃ | CH₂OCH₃ |
| A-182 | 2-C₃H₅ | CH₂OCH₃ |
| A-183 | 2-C(=NOCH₃)CH₃ | CH₂OCH₃ |
| A-184 | 2-CN | CH₂OCH₃ |
| A-185 | 3-F | CH₂OCH₃ |
| A-186 | 3-Cl | CH₂OCH₃ |
| A-187 | 3-Br | CH₂OCH₃ |
| A-188 | 3-CH₃ | CH₂OCH₃ |
| A-189 | 3-CHF₂ | CH₂OCH₃ |
| A-190 | 3-CF₃ | CH₂OCH₃ |
| A-191 | 3-OCH₃ | CH₂OCH₃ |
| A-192 | 3-OCHF₂ | CH₂OCH₃ |
| A-193 | 3-OCF₃ | CH₂OCH₃ |
| A-194 | 3-C₂H₅ | CH₂OCH₃ |
| A-195 | 3-CH₂CF₃ | CH₂OCH₃ |
| A-196 | 3-CH=CH₂ | CH₂OCH₃ |
| A-197 | 3-C₆H₅ | CH₂OCH₃ |
| A-198 | 3-C≡CH | CH₂OCH₃ |
| A-199 | 3-C≡CCH₃ | CH₂OCH₃ |
| A-200 | 3-C₃H₅ | CH₂OCH₃ |
| A-201 | 3-C(=NOCH₃)CH₃ | CH₂OCH₃ |
| A-202 | 3-CN | CH₂OCH₃ |
| A-203 | 4-F | CH₂OCH₃ |
| A-204 | 4-Cl | CH₂OCH₃ |
| A-205 | 4-Br | CH₂OCH₃ |
| A-206 | 4-CH₃ | CH₂OCH₃ |
| A-207 | 4-CHF₂ | CH₂OCH₃ |
| A-208 | 4-CF₃ | CH₂OCH₃ |
| A-209 | 4-OCH₃ | CH₂OCH₃ |
| A-210 | 4-OCHF₂ | CH₂OCH₃ |
| A-211 | 4-OCF₃ | CH₂OCH₃ |
| A-212 | 4-C₂H₅ | CH₂OCH₃ |
| A-213 | 4-CH₂CF₃ | CH₂OCH₃ |
| A-214 | 4-CH=CH₂ | CH₂OCH₃ |
| A-215 | 4-C₆H₅ | CH₂OCH₃ |
| A-216 | 4-C≡CH | CH₂OCH₃ |
| A-217 | 4-C≡CCH₃ | CH₂OCH₃ |
| A-218 | 4-C₃H₅ | CH₂OCH₃ |
| A-219 | 4-C(=NOCH₃)CH₃ | CH₂OCH₃ |
| A-220 | 4-CN | CH₂OCH₃ |
| A-221 | - | CH₂-C(=NOCH₃)CH₃ |
| A-222 | 2-F | CH₂-C(=NOCH₃)CH₃ |
| A-223 | 2-Cl | CH₂-C(=NOCH₃)CH₃ |
| A-224 | 2-Br | CH₂-C(=NOCH₃)CH₃ |
| A-225 | 2-CH₃ | CH₂-C(=NOCH₃)CH₃ |
| A-226 | 2-CHF₂ | CH₂-C(=NOCH₃)CH₃ |
| A-227 | 2-CF₃ | CH₂-C(=NOCH₃)CH₃ |
| A-228 | 2-OCH₃ | CH₂-C(=NOCH₃)CH₃ |
| A-229 | 2-OCHF₂ | CH₂-C(=NOCH₃)CH₃ |
| A-230 | 2-OCF₃ | CH₂-C(=NOCH₃)CH₃ |
| A-231 | 2-C₂H₅ | CH₂-C(=NOCH₃)CH₃ |
| A-232 | 2-CH₂CF₃ | CH₂-C(=NOCH₃)CH₃ |
| A-233 | 2-CH=CH₂ | CH₂-C(=NOCH₃)CH₃ |
| A-234 | 2-C₆H₅ | CH₂-C(=NOCH₃)CH₃ |
| A-235 | 2-C≡CH | CH₂-C(=NOCH₃)CH₃ |
| A-236 | 2-C≡CCH₃ | CH₂-C(=NOCH₃)CH₃ |
| A-237 | 2-C₃H₅ | CH₂-C(=NOCH₃)CH₃ |
| A-238 | 2-C(=NOCH₃)CH₃ | CH₂-C(=NOCH₃)CH₃ |
| A-239 | 2-CN | CH₂-C(=NOCH₃)CH₃ |
| A-240 | 3-F | CH₂-C(=NOCH₃)CH₃ |
| A-241 | 3-Cl | CH₂-C(=NOCH₃)CH₃ |
| A-242 | 3-Br | CH₂-C(=NOCH₃)CH₃ |
| A-243 | 3-CH₃ | CH₂-C(=NOCH₃)CH₃ |
| A-244 | 3-CHF₂ | CH₂-C(=NOCH₃)CH₃ |
| A-245 | 3-CF₃ | CH₂-C(=NOCH₃)CH₃ |
| A-246 | 3-OCH₃ | CH₂-C(=NOCH₃)CH₃ |
| A-247 | 3-OCHF₂ | CH₂-C(=NOCH₃)CH₃ |
| A-248 | 3-OCF₃ | CH₂-C(=NOCH₃)CH₃ |
| A-249 | 3-C₂H₅ | CH₂-C(=NOCH₃)CH₃ |
| A-250 | 3-CH₂CF₃ | CH₂-C(=NOCH₃)CH₃ |
| A-251 | 3-CH=CH₂ | CH₂-C(=NOCH₃)CH₃ |
| A-252 | 3-C₆H₅ | CH₂-C(=NOCH₃)CH₃ |
| A-253 | 3-C≡CH | CH₂-C(=NOCH₃)CH₃ |
| A-254 | 3-C≡CCH₃ | CH₂-C(=NOCH₃)CH₃ |
| A-255 | 3-C₃H₅ | CH₂-C(=NOCH₃)CH₃ |
| A-256 | 3-C(=NOCH₃)CH₃ | CH₂-C(=NOCH₃)CH₃ |
| A-257 | 3-CN | CH₂-C(=NOCH₃)CH₃ |
| A-258 | 4-F | CH₂-C(=NOCH₃)CH₃ |
| A-259 | 4-Cl | CH₂-C(=NOCH₃)CH₃ |
| A-260 | 4-Br | CH₂-C(=NOCH₃)CH₃ |
| A-261 | 4-CH₃ | CH₂-C(=NOCH₃)CH₃ |
| A-262 | 4-CHF₂ | CH₂-C(=NOCH₃)CH₃ |
| A-263 | 4-CF₃ | CH₂-C(=NOCH₃)CH₃ |
| A-264 | 4-OCH₃ | CH₂-C(=NOCH₃)CH₃ |
| A-265 | 4-OCHF₂ | CH₂-C(=NOCH₃)CH₃ |
| A-266 | 4-OCF₃ | CH₂-C(=NOCH₃)CH₃ |
| A-267 | 4-C₂H₅ | CH₂-C(=NOCH₃)CH₃ |
| A-268 | 4-CH₂CF₃ | CH₂-C(=NOCH₃)CH₃ |
| A-269 | 4-CH=CH₂ | CH₂-C(=NOCH₃)CH₃ |
| A-270 | 4-C₆H₅ | CH₂-C(=NOCH₃)CH₃ |
| A-271 | 4-C≡CH | CH₂-C(=NOCH₃)CH₃ |
| A-272 | 4-C≡CCH₃ | CH₂-C(=NOCH₃)CH₃ |
| A-273 | 4-C₃H₅ | CH₂-C(=NOCH₃)CH₃ |
| A-274 | 4-C(=NOCH₃)CH₃ | CH₂-C(=NOCH₃)CH₃ |
| A-275 | 4-CN | CH₂-C(=NOCH₃)CH₃ |
| A-276 | - | CHF₂ |
| A-277 | 2-F | CHF₂ |
| A-278 | 2-Cl | CHF₂ |
| A-279 | 2-Br | CHF₂ |
| A-280 | 2-CH₃ | CHF₂ |
| A-281 | 2-CHF₂ | CHF₂ |
| A-282 | 2-CF₃ | CHF₂ |
| A-283 | 2-OCH₃ | CHF₂ |
| A-284 | 2-OCHF₂ | CHF₂ |
| A-285 | 2-OCF₃ | CHF₂ |
| A-286 | 2-C₂H₅ | CHF₂ |
| A-287 | 2-CH₂CF₃ | CHF₂ |
| A-288 | 2-CH=CH₂ | CHF₂ |
| A-289 | 2-C₆H₅ | CHF₂ |
| A-290 | 2-C≡CH | CHF₂ |
| A-291 | 2-C≡CCH₃ | CHF₂ |
| A-292 | 2-C₃H₅ | CHF₂ |
| A-293 | 2-C(=NOCH₃)CH₃ | CHF₂ |
| A-294 | 2-CN | CHF₂ |
| A-295 | 3-F | CHF₂ |
| A-296 | 3-Cl | CHF₂ |
| A-297 | 3-Br | CHF₂ |
| A-298 | 3-CH₃ | CHF₂ |
| A-299 | 3-CHF₂ | CHF₂ |
| A-300 | 3-CF₃ | CHF₂ |
| A-301 | 3-OCH₃ | CHF₂ |
| A-302 | 3-OCHF₂ | CHF₂ |
| A-303 | 3-OCF₃ | CHF₂ |
| A-304 | 3-C₂H₅ | CHF₂ |
| A-305 | 3-CH₂CF₃ | CHF₂ |
| A-306 | 3-CH=CH₂ | CHF₂ |
| A-307 | 3-C₆H₅ | CHF₂ |
| A-308 | 3-C≡CH | CHF₂ |
| A-309 | 3-C≡CCH₃ | CHF₂ |
| A-310 | 3-C₃H₅ | CHF₂ |
| A-311 | 3-C(=NOCH₃)CH₃ | CHF₂ |
| A-312 | 3-CN | CHF₂ |
| A-313 | 4-F | CHF₂ |
| A-314 | 4-Cl | CHF₂ |
| A-315 | 4-Br | CHF₂ |
| A-316 | 4-CH₃ | CHF₂ |
| A-317 | 4-CHF₂ | CHF₂ |
| A-318 | 4-CF₃ | CHF₂ |
| A-319 | 4-OCH₃ | CHF₂ |
| A-320 | 4-OCHF₂ | CHF₂ |
| A-321 | 4-OCF₃ | CHF₂ |
| A-322 | 4-C₂H₅ | CHF₂ |
| A-323 | 4-CH₂CF₃ | CHF₂ |
| A-324 | 4-CH=CH₂ | CHF₂ |
| A-325 | 4-C₆H₅ | CHF₂ |
| A-326 | 4-C≡CH | CHF₂ |
| A-327 | 4-C≡CCH₃ | CHF₂ |
| A-328 | 4-C₃H₅ | CHF₂ |
| A-329 | 4-C(=NOCH₃)CH₃ | CHF₂ |
| A-330 | 4-CN | CHF₂ |
| A-331 | - | CH₂C₃H₅ |
| A-332 | 2-F | CH₂C₃H₅ |
| A-333 | 2-Cl | CH₂C₃H₅ |
| A-334 | 2-Br | CH₂C₃H₅ |
| A-335 | 2-CH₃ | CH₂C₃H₅ |
| A-336 | 2-CHF₂ | CH₂C₃H₅ |
| A-337 | 2-CF₃ | CH₂C₃H₅ |
| A-338 | 2-OCH₃ | CH₂C₃H₅ |
| A-339 | 2-OCHF₂ | CH₂C₃H₅ |
| A-340 | 2-OCF₃ | CH₂C₃H₅ |
| A-341 | 2-C₂H₅ | CH₂C₃H₅ |
| A-342 | 2-CH₂CF₃ | CH₂C₃H₅ |
| A-343 | 2-CH=CH₂ | CH₂C₃H₅ |
| A-344 | 2-C₆H₅ | CH₂C₃H₅ |
| A-345 | 2-C≡CH | CH₂C₃H₅ |
| A-346 | 2-C≡CCH₃ | CH₂C₃H₅ |
| A-347 | 2-C₃H₅ | CH₂C₃H₅ |
| A-348 | 2-C(=NOCH₃)CH₃ | CH₂C₃H₅ |
| A-349 | 2-CN | CH₂C₃H₅ |
| A-350 | 3-F | CH₂C₃H₅ |
| A-351 | 3-Cl | CH₂C₃H₅ |
| A-352 | 3-Br | CH₂C₃H₅ |
| A-353 | 3-CH₃ | CH₂C₃H₅ |
| A-354 | 3-CHF₂ | CH₂C₃H₅ |
| A-355 | 3-CF₃ | CH₂C₃H₅ |
| A-356 | 3-OCH₃ | CH₂C₃H₅ |
| A-357 | 3-OCHF₂ | CH₂C₃H₅ |
| A-358 | 3-OCF₃ | CH₂C₃H₅ |
| A-359 | 3-C₂H₅ | CH₂C₃H₅ |
| A-360 | 3-CH₂CF₃ | CH₂C₃H₅ |
| A-361 | 3-CH=CH₂ | CH₂C₃H₅ |
| A-362 | 3-C₆H₅ | CH₂C₃H₅ |
| A-363 | 3-C≡CH | CH₂C₃H₅ |
| A-364 | 3-C≡CCH₃ | CH₂C₃H₅ |
| A-365 | 3-C₃H₅ | CH₂C₃H₅ |
| A-366 | 3-C(=NOCH₃)CH₃ | CH₂C₃H₅ |
| A-367 | 3-CN | CH₂C₃H₅ |
| A-368 | 4-F | CH₂C₃H₅ |
| A-369 | 4-Cl | CH₂C₃H₅ |
| A-370 | 4-Br | CH₂C₃H₅ |
| A-371 | 4-CH₃ | CH₂C₃H₅ |
| A-372 | 4-CHF₂ | CH₂C₃H₅ |
| A-373 | 4-CF₃ | CH₂C₃H₅ |
| A-374 | 4-OCH₃ | CH₂C₃H₅ |
| A-375 | 4-OCHF₂ | CH₂C₃H₅ |
| A-376 | 4-OCF₃ | CH₂C₃H₅ |
| A-377 | 4-C₂H₅ | CH₂C₃H₅ |
| A-378 | 4-CH₂CF₃ | CH₂C₃H₅ |
| A-379 | 4-CH=CH₂ | CH₂C₃H₅ |
| A-380 | 4-C₆H₅ | CH₂C₃H₅ |
| A-381 | 4-C≡CH | CH₂C₃H₅ |
| A-382 | 4-C≡CCH₃ | CH₂C₃H₅ |
| A-383 | 4-C₃H₅ | CH₂C₃H₅ |
| A-384 | 4-C(=NOCH₃)CH₃ | CH₂C₃H₅ |
| A-385 | 4-CN | CH₂C₃H₅ |
| A-386 | 2-F, 3-F | CH₃ |
| A-387 | 2-F, 4-F | CH₃ |
| A-388 | 2-F, 5-F | CH₃ |
| A-389 | 3-F, 4-F | CH₃ |
| A-390 | 3-F, 5-F | CH₃ |
| A-391 | 2-Cl, 3-Cl | CH₃ |
| A-392 | 2-Cl, 4-Cl | CH₃ |
| A-393 | 2-Cl, 5-Cl | CH₃ |
| A-394 | 3-Cl, 4-Cl | CH₃ |
| A-395 | 3-Cl, 5-Cl | CH₃ |
| A-396 | 2-CH₃, 3-CH₃ | CH₃ |
| A-397 | 2-CH₃, 4-CH₃ | CH₃ |
| A-398 | 2-CH₃, 5-CH₃ | CH₃ |
| A-399 | 3-CH₃, 4-CH₃ | CH₃ |
| A-400 | 3-CH₃, 5-CH₃ | CH₃ |
| A-401 | 2-OCH₃, 3-OCH₃ | CH₃ |
| A-402 | 2-OCH₃, 4-OCH₃ | CH₃ |
| A-403 | 2-OCH₃, 5-OCH₃ | CH₃ |
| A-404 | 3-OCH₃, 4-OCH₃ | CH₃ |
| A-405 | 3-OCH₃, 5-OCH₃ | CH₃ |
| A-406 | 2-CF₃, 3-CF₃ | CH₃ |
| A-407 | 2-CF₃, 4-CF₃ | CH₃ |
| A-408 | 2-CF₃, 5-CF₃ | CH₃ |
| A-409 | 3-CF₃, 4-CF₃ | CH₃ |
| A-410 | 3-CF₃, 5-CF₃ | CH₃ |
| A-411 | 2-OCF₃, 3-OCF₃ | CH₃ |
| A-412 | 2-OCF₃, 4-OCF₃ | CH₃ |
| A-413 | 2-OCF₃, 5-OCF₃ | CH₃ |
| A-414 | 3-OCF₃, 4-OCF₃ | CH₃ |
| A-415 | 3-OCF₃, 5-OCF₃ | CH₃ |
| A-416 | 2-F, 3-Cl | CH₃ |
| A-417 | 2-F, 4-Cl | CH₃ |
| A-418 | 2-F, 5-Cl | CH₃ |
| A-419 | 3-F, 4-Cl | CH₃ |
| A-420 | 3-F, 5-Cl | CH₃ |
| A-421 | 2-CH₃, 3-Cl | CH₃ |
| A-422 | 2-CH₃, 4-Cl | CH₃ |
| A-423 | 2-CH₃, 5-Cl | CH₃ |
| A-424 | 3-CH₃, 4-Cl | CH₃ |
| A-425 | 3-CH₃, 5-Cl | CH₃ |
| A-426 | 2-OCH₃, 3-Cl | CH₃ |
| A-427 | 2-OCH₃, 4-Cl | CH₃ |
| A-428 | 2-OCH₃, 5-Cl | CH₃ |
| A-429 | 3-OCH₃, 4-Cl | CH₃ |
| A-430 | 3-OCH₃, 5-Cl | CH₃ |
| A-431 | 2-CF₃, 3-Cl | CH₃ |
| A-432 | 2-CF₃, 4-Cl | CH₃ |
| A-433 | 2-CF₃, 5-Cl | CH₃ |
| A-434 | 3-CF₃, 4-Cl | CH₃ |
| A-435 | 3-CF₃, 5-Cl | CH₃ |
| A-436 | 2-OCF₃, 3-Cl | CH₃ |
| A-437 | 2-OCF₃, 4-Cl | CH₃ |
| A-438 | 2-OCF₃, 5-Cl | CH₃ |
| A-439 | 3-OCF₃, 4-Cl | CH₃ |
| A-440 | 3-OCF₃, 5-Cl | CH₃ |
| A-441 | 2-F, 3-CH₃ | CH₃ |
| A-442 | 2-F, 4-CH₃ | CH₃ |
| A-443 | 2-F, 5-CH₃ | CH₃ |
| A-444 | 3-F, 4-CH₃ | CH₃ |
| A-445 | 3-F, 5-CH₃ | CH₃ |
| A-446 | 2-Cl, 3-CH₃ | CH₃ |
| A-447 | 2-Cl, 4-CH₃ | CH₃ |
| A-448 | 2-Cl, 5-CH₃ | CH₃ |
| A-449 | 3-Cl, 4-CH₃ | CH₃ |
| A-450 | 3-Cl, 5-CH₃ | CH₃ |
| A-451 | 2-OCH₃, 3-CH₃ | CH₃ |
| A-452 | 2-OCH₃, 4-CH₃ | CH₃ |
| A-453 | 2-OCH₃, 5-CH₃ | CH₃ |
| A-454 | 3-OCH₃, 4-CH₃ | CH₃ |
| A-455 | 3-OCH₃, 5-CH₃ | CH₃ |
| A-456 | 2-CF₃, 3-CH₃ | CH₃ |
| A-457 | 2-CF₃, 4-CH₃ | CH₃ |
| A-458 | 2-CF₃, 5-CH₃ | CH₃ |
| A-459 | 3-CF₃, 4-CH₃ | CH₃ |
| A-460 | 3-CF₃, 5-CH₃ | CH₃ |
| A-461 | 2-OCF₃, 3-CH₃ | CH₃ |
| A-462 | 2-OCF₃, 4-CH₃ | CH₃ |
| A-463 | 2-OCF₃, 5-CH₃ | CH₃ |
| A-464 | 3-OCF₃, 4-CH₃ | CH₃ |
| A-465 | 3-OCF₃, 5-CH₃ | CH₃ |
| A-466 | 2-F, 3-OCH₃ | CH₃ |
| A-467 | 2-F, 4-OCH₃ | CH₃ |
| A-468 | 2-F, 5-OCH₃ | CH₃ |
| A-469 | 3-F, 4-OCH₃ | CH₃ |
| A-470 | 3-F, 5-OCH₃ | CH₃ |
| A-471 | 2-Cl, 3-OCH₃ | CH₃ |
| A-472 | 2-Cl, 4-OCH₃ | CH₃ |
| A-473 | 2-Cl, 5-OCH₃ | CH₃ |
| A-474 | 3-Cl, 4-OCH₃ | CH₃ |
| A-475 | 3-Cl, 5-OCH₃ | CH₃ |
| A-476 | 2-CH₃, 3-OCH₃ | CH₃ |
| A-477 | 2-CH₃, 4-OCH₃ | CH₃ |
| A-478 | 2-CH₃, 5-OCH₃ | CH₃ |
| A-479 | 3-CH₃, 4-OCH₃ | CH₃ |
| A-480 | 3-CH₃, 5-OCH₃ | CH₃ |
| A-481 | 2-CF₃, 3-OCH₃ | CH₃ |
| A-482 | 2-CF₃, 4-OCH₃ | CH₃ |
| A-483 | 2-CF₃, 5-OCH₃ | CH₃ |
| A-484 | 3-CF₃, 4-OCH₃ | CH₃ |
| A-485 | 3-CF₃, 5-OCH₃ | CH₃ |
| A-486 | 2-OCF₃, 3-OCH₃ | CH₃ |
| A-487 | 2-OCF₃, 4-OCH₃ | CH₃ |
| A-488 | 2-OCF₃, 5-OCH₃ | CH₃ |
| A-489 | 3-OCF₃, 4-OCH₃ | CH₃ |
| A-490 | 3-OCF₃, 5-OCH₃ | CH₃ |
| A-491 | 2-F, 3-CF₃ | CH₃ |
| A-492 | 2-F, 4-CF₃ | CH₃ |
| A-493 | 2-F, 5-CF₃ | CH₃ |
| A-494 | 3-F, 4-CF₃ | CH₃ |
| A-495 | 3-F, 5-CF₃ | CH₃ |
| A-496 | 2-Cl, 3-CF₃ | CH₃ |
| A-497 | 2-Cl, 4-CF₃ | CH₃ |
| A-498 | 2-Cl, 5-CF₃ | CH₃ |
| A-499 | 3-Cl, 4-CF₃ | CH₃ |
| A-500 | 3-Cl, 5-CF₃ | CH₃ |
| A-501 | 2-CH₃, 3-CF₃ | CH₃ |
| A-502 | 2-CH₃, 4-CF₃ | CH₃ |
| A-503 | 2-CH₃, 5-CF₃ | CH₃ |
| A-504 | 3-CH₃, 4-CF₃ | CH₃ |
| A-505 | 3-CH₃, 5-CF₃ | CH₃ |
| A-506 | 2-OCH₃, 3-CF₃ | CH₃ |
| A-507 | 2-OCH₃, 4-CF₃ | CH₃ |
| A-508 | 2-OCH₃, 5-CF₃ | CH₃ |
| A-509 | 3-OCH₃, 4-CF₃ | CH₃ |
| A-510 | 3-OCH₃, 5-CF₃ | CH₃ |
| A-511 | 2-OCF₃, 3-CF₃ | CH₃ |
| A-512 | 2-OCF₃, 4-CF₃ | CH₃ |
| A-513 | 2-OCF₃, 5-CF₃ | CH₃ |
| A-514 | 3-OCF₃, 4-CF₃ | CH₃ |
| A-515 | 3-OCF₃, 5-CF₃ | CH₃ |
| A-516 | 2-F, 3-OCF₃ | CH₃ |
| A-517 | 2-F, 4-OCF₃ | CH₃ |
| A-518 | 2-F, 5-OCF₃ | CH₃ |
| A-519 | 3-F, 4-OCF₃ | CH₃ |
| A-520 | 3-F, 5-OCF₃ | CH₃ |
| A-521 | 2-Cl, 3-OCF₃ | CH₃ |
| A-522 | 2-Cl, 4-OCF₃ | CH₃ |
| A-523 | 2-Cl, 5-OCF₃ | CH₃ |
| A-524 | 3-Cl, 4-OCF₃ | CH₃ |
| A-525 | 3-Cl, 5-OCF₃ | CH₃ |
| A-526 | 2-CH₃, 3-OCF₃ | CH₃ |
| A-527 | 2-CH₃, 4-OCF₃ | CH₃ |
| A-528 | 2-CH₃, 5-OCF₃ | CH₃ |
| A-529 | 3-CH₃, 4-OCF₃ | CH₃ |
| A-530 | 3-CH₃, 5-OCF₃ | CH₃ |
| A-531 | 2-OCH₃, 3-OCF₃ | CH₃ |
| A-532 | 2-OCH₃, 4-OCF₃ | CH₃ |
| A-533 | 2-OCH₃, 5-OCF₃ | CH₃ |
| A-534 | 3-OCH₃, 4-OCF₃ | CH₃ |
| A-535 | 3-OCH₃, 5-OCF₃ | CH₃ |
| A-536 | 2-CF₃, 3-OCF₃ | CH₃ |
| A-537 | 2-CF₃, 4-OCF₃ | CH₃ |
| A-538 | 2-CF₃, 5-OCF₃ | CH₃ |
| A-539 | 3-CF₃, 4-OCF₃ | CH₃ |
| A-540 | 3-CF₃, 5-OCF₃ | CH₃ |
| A-541 | 3,4,5-F₃* | CH₃ |
| A-542 | 2,4,6-F₃ | CH₃ |
| A-543 | 3,4,5-Cl₃ | CH₃ |
| A-544 | 2,4,6-Cl₃ | CH₃ |
| A-545 | 3,4,5-(CH₃)₃ | CH₃ |
| A-546 | 2,4,6-(CH₃)₃ | CH₃ |
| A-547 | 3,4,5-(OCH₃)₃ | CH₃ |
| A-548 | 2,4,6-(OCH₃)₃ | CH₃ |
| A-549 | 3,4,5-(CF₃)₃ | CH₃ |
| A-550 | 2,4,6-(CF₃)₃ | CH₃ |
| A-551 | 3,4,5-(OCF₃)₃ | CH₃ |
| A-552 | 2,4,6-(OCF₃)₃ | CH₃ |

| | | |
|---|---|---|
| * refers to 3 fluoro at positions 3,4 and 5. | | |

The compounds can be obtained by various routes in analogy to prior art processes known (e.g EP 463488) and, advantageously, by the synthesis shown in the following Schemes 1 to 4 and in the experimental part of this application.

A suitable method to prepare compounds I is illustrated in Scheme 1.

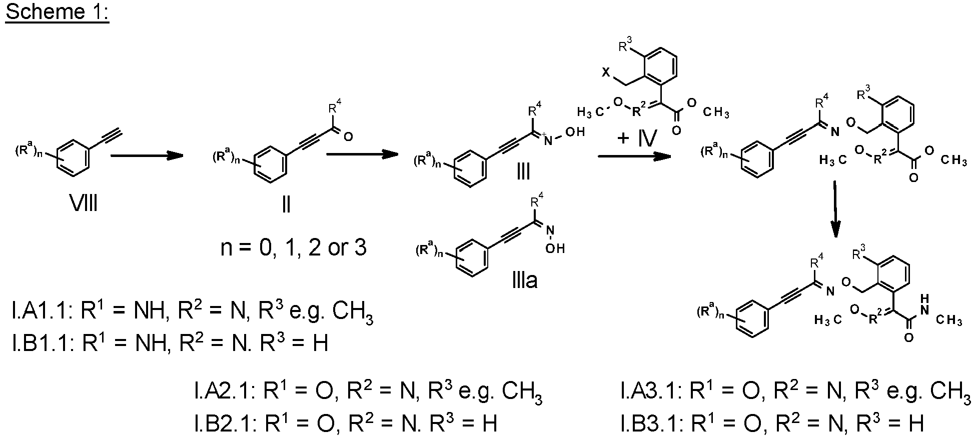

It starts with the formation of a ketone II from the corresponding acetylene compound VIII. Acetylenic proton is first abstracted by a base such as n-butyl lithium in a solvent such as tetrahydrofuran (THF) or 2-methyl-THF at reaction temperatures of -78 °C followed by quenching it with an electrophile such as ethyl acetate in the presence of BF₃-diethyl etherate at -78 °C. The conversion of the ketone II to the corresponding oxime is performed using hydxroxylamine hydrochloride and a base such as pyridine or sodium acetate in polar solvents such as methanol-water mixture at reaction temperatures of about 10 to 25 °C for 2 to 4 h, preferably at about 15 °C. The reaction provides a mixture of E-oxime III and Z-oxime IIIa. The E/Z oxime mixture (III and IIIa) is subjected to a reaction with the intermediate IV, wherein X is a leaving group such as halogen, toluene-methanesulfonates, preferably X is Cl or Br, under basic conditions using bases such as sodium hydride, cesium carbonate or potassium carbonate and using an organic solvent such as dimethyl formamide (DMF) or acetonitrile, preferably cesium carbonate as base and acetonitrile (AcN) as solvent at room temperature (RT) of about 24 °C for 12h. The resulting ester compound I.2 wherein R¹ is O can be converted to the amide of formula 1.1 wherein R¹ is NH by reaction with methyl amine (preferably 40% aq. solution) using THF as solvent at RT.

A general method for preparation of intermediate IV is shown in Scheme 2.

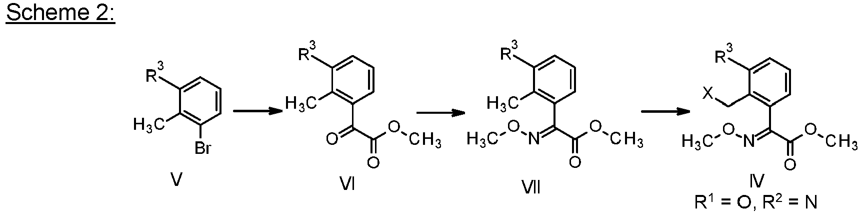

Compound VI can be obtained from compound V by lithium-halogen exchange or by generating Grignard reagent and further reaction with dimethyl oxalate or chloromethyl oxalate in presence of a solvent. The preferred solvent is THF or 2-methyl-THF and the temperature can be between -70 and -78 °C. Conversion of intermediate VI to intermediate VII can be achieved using N-methylhydroxylamine hydrochloride and a base such as pyridine or sodium acetate in polar solvents such as methanol. The reaction temperature is preferably about 65 °C. An E/Z mixture is usually obtained These isomers can be separated by purification techniques known in art (e.g. column chromatography, crystallization). Bromination of intermediate VII provides the desired intermediate compounds IV, wherein R¹ is O and R² = N and X is Br. This reaction of intermediate VII with N-bromosuccinimide is carried out in solvents such as carbon tetrachloride, chlorobenzene and acetonitrile, using radical initiators such as 1,1'-azobis(cyclo-hexanecarbonitrile) or azobisisobutyronitrile at temperatures of about 70 to 100 °C. The preferred radical initiator is 1,1'-azobis(cyclohexanecarbonitrile), preferred solvent chlorobenzene and preferred temperature 80 °C.

The synthesis of compounds containing different substituents R³ follows similar sequence as in Scheme 2, wherein R³ is bromo. Coupling of intermediate III with intermediate IV, wherein R³ is bromo, provides compounds I as described above. Using standard chemical reactions, such as Suzuki or Stille reaction, the bromo group can be converted e.g. to other R³ substituents such as cycloalkyl, alkoxy and alkenyl. Additional transformations e.g. of ethenyl provide compounds I with other R³ substituents such as ethyl, CN and haloalkyl.

Preparation of ketones II can also be carried out using other known methods (Scheme 3).

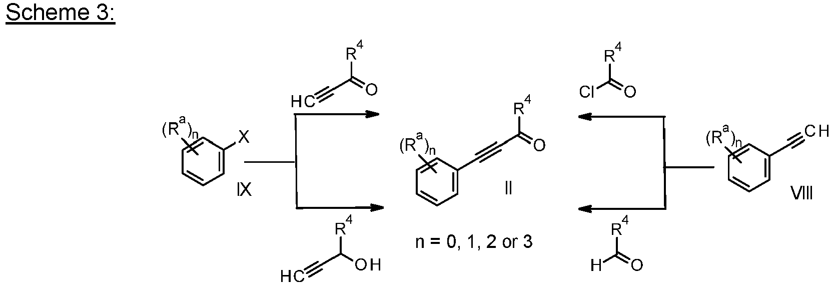

Palladium and/or copper-catalyzed cross couplings of terminal acetylenic compounds VIII with acid chlorides are usually applied (J. Org. Chem. 2004, 69, 1615). Another possibility is by formation of base-catalyzed alumination of terminal alkynes VIII followed by reaction of it with acid chlorides (J. Org. Chem. 2005, 70, 6126-6128). Alternatively, ketones II can be obtained from acetylene VIII by abstraction of acetylenic proton followed by quenching it with an aldehyde and subsequent oxidation of alcohol (Org. Biomol. Chem. 2018, 16, 6659). Ketone II can also be obtained from precursors IX, wherein X is halogen preferably iodine b palladium and copper-catalyzed cross couplings of aryl halide IX with terminal alkynones (Chem. Cat. Chem. 2015, 7, 3266) or with alkynyl alcohol followed by oxidation of alcohol (Adv. Synth. Cat. 2017, 22, 4062).

Compounds VIII are generally commercially available, otherwise accessible using methods known in prior art (Org. Lett. 2019, 21, 3990).

Another general method to prepare the compounds I is depicted in Scheme 4.

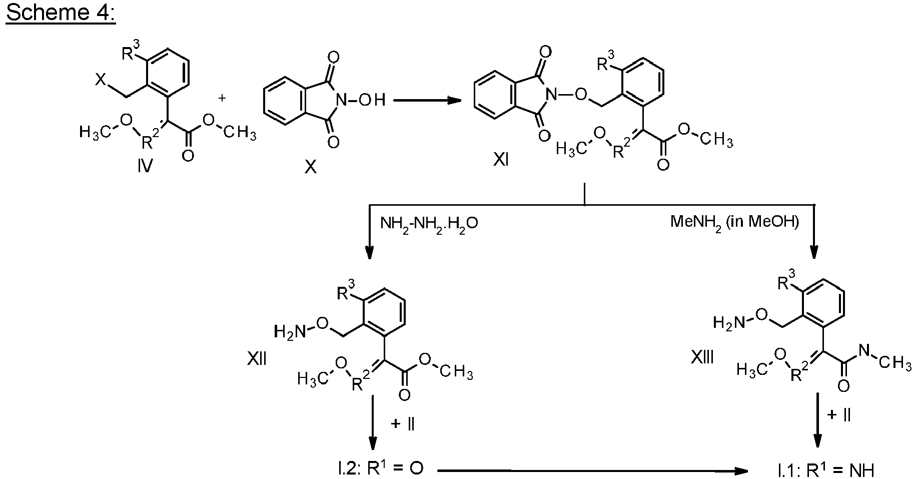

Intermediate IV is reacted with N-hydroxysuccimide X, using a base such as triethylamine in DMF. The reaction temperature is usually 50 to 70 °C preferably about 70 °C. Conversion to the corresponding O-benzylhydroxyl amine XII, is achieved through removal of the phthalimide group, preferably using hydrazine hydrate in methanol as solvent at about 25 °C. Alternatively, removal of the phthalimide group using methyl amine in methanol as solvent at about 25 °C can provide intermediate XIII. Intermediate XII and XIII, respectively can be condensed with ketones II using acetic acid or pyridine in methanol as solvent at about 50 to 65 °C. Alternatively, the condensation can also be carried out with titanium (IV) ethoxide (Ti(OEt)₄) using THF as solvent at about 70 °C. The desired product is usually accompanied by an undesired isomer, which can be removed e.g by column chromatography, crystallization.

The Schemes 1 to 4 presented above describe the synthesis of compounds I wherein R³ is in ortho position to the methyl oxime side chain but can also be applied likewise to corresponding compounds wherein R³ is at different position of the phenyl ring.

The compounds I and the compositions thereof, respectively, are suitable as fungicides effective against a broad spectrum of phytopathogenic fungi, including soil-borne fungi, in particular from the classes of Plasmodiophoromycetes, Peronosporomycetes (syn. Oomycetes), Chytridiomycetes, Zygomycetes, Ascomycetes, Basidiomycetes, and Deuteromycetes (syn. Fungi imperfecti). They can be used in crop protection as foliar fungicides, fungicides for seed dressing, and soil fungicides.

The compounds I and the compositions thereof are preferably useful in the control of phytopathogenic fungi on various cultivated plants, such as cereals, e. g. wheat, rye, barley, triticale, oats, or rice; beet, fruits, leguminous plants such as soybean, oil plants, cucurbits, fiber plants, citrus fruits, vegetables, lauraceous plants, energy and raw material plants, corn; tobacco; nuts; coffee; tea; bananas; vines (table grapes and grape juice grape vines); natural rubber plants; or ornamental and forestry plants; on the plant propagation material, such as seeds; and on the crop material of these plants.

According to the invention all of the above cultivated plants are understood to comprise all species, subspecies, variants, varieties and/or hybrids which belong to the respective cultivated plants, including but not limited to winter and spring varieties, in particular in cereals such as wheat and barley, as well as oilseed rape, e.g. winter wheat, spring wheat, winter barley etc.

Corn is also known as Indian corn or maize (Zea *mays)* which comprises all kinds of corn such as field corn and sweet corn. According to the invention all soybean cultivars or varieties are comprised, in particular indeterminate and determinate cultivars or varieties.

The term "cultivated plants" is to be understood as including plants which have been modified by mutagenesis or genetic engineering to provide a new trait to a plant or to modify an already present trait.

The compounds I and compositions thereof, respectively, are particularly suitable for controlling the following causal agents of plant diseases: rusts on soybean and cereals (e.g. *Phakopsora pachyrhizi* and P. *meibomiae* on soybean; *Puccinia tritici* and P. *striiformis* on wheat); molds on specialty crops, soybean, oil seed rape and sunflowers (e.g. *Botrytis cinerea* on strawberries and vines, *Sclerotinia sclerotiorum, S. minor* and *S. rolfsii* on oil seed rape, sunflowers and soybean); Fusarium diseases on cereals (e.g. *Fusarium culmorum* and F. *graminearum* on wheat); downy mildews on specialty crops (e.g. *Plasmopara viticola* on vines, *Phytophthora infestans* on potatoes); powdery mildews on specialty crops and cereals (e.g. *Uncinula necator* on vines, *Erysiphe* spp. on various specialty crops, *Blumeria graminis* on cereals); and leaf spots on cereals, soybean and corn (e.g. *Septoria tritici* and S. *nodorum* on cereals, S. *glycines* on soybean, *Cercospora* spp. on corn and soybean).

The compounds I and compositions thereof, respectively, are particularly suitable for for combating phytopathogenic fungi containing an amino acid substitution F129L in the mitochondrial cytochrome b protein conferring resistance to Qo inhibitors.

The mutation F129L in the cytochrome b (cytb, also referred to as cob) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the cytochrome b gene resulting in a single amino acid substitution in the position 129 from F (phenylalanine) to L (leucine) (F129L) in the cytochrome b protein (Cytb). In the present invention, the mutation F129L in the cytochrome b gene shall be understood to be a single amino acid substitution in the position 129 from F (phenylalanine) to L (leucine) (F129L) in the cytochrome b protein.

Many other phytopathogenic fungi acquired the F129L mutation in the cytochrome b gene conferring resistance to Qo inhibitors, such as rusts, in particular soybean rust *(Phakopsora pachyrhizi* and *Phakopsora meibromiae)* as well as fungi from the genera Alternaria, Pyrenophora and Rhizoctonia.

Preferred fungal species are *Alternaria solani, Phakopsora pachyrhizi, Phakopsora meibromiae, Pyrenophora teres, Pyrenophora tritici-repentis* and *Rhizoctonia solani;* in particular *Phakopsora pachyrhizi.*

In one aspect, the present invention relates to the method of protecting plants susceptible to and/or under attack by phytopathogenic fungi containing an amino acid substitution F129L in the mitochondrial cytochrome b protein conferring resistance to Qo inhibitors, which method comprises applying to said plants, treating plant propagation material of said plants with, and/or applying to said phytopathogenic fungi, at least one compound of formula I or a composition comprising at least one compound of formula I.

According to another embodiment, the method for combating phytopathogenic fungi, comprises: a) identifying the phytopathogenic fungi containing an amino acid substitution F129L in the mitochondrial cytochrome b protein conferring resistance to Qo inhibitors, or the materials, plants, the soil or seeds that are at risk of being diseased from phytopathogenic fungi as defined herein, and b) treating said fungi or the materials, plants, the soil or plant propagation material with an effective amount of at least one compound of formula I, or a composition comprising it thereof.

The term "phytopathogenic fungi an amino acid substitution F129L in the mitochondrial cytochrome b protein conferring resistance to Qo inhibitors" is to be understood that at least 10% of the fungal isolates to be controlled contain a such F129L substitution in the mitochondrial cytochrome b protein conferring resistance to Qo inhibitors, preferably at least 30%, more preferably at least 50%, even more preferably at at least 75% of the fungi, most preferably between 90 and 100%; in particular between 95 and 100%.

The compounds I and compositions thereof, respectively, are also suitable for controlling harmful microorganisms in the protection of stored products or harvest, and in the protection of materials.

The compounds I are employed as such or in form of compositions by treating the fungi, the plants, plant propagation materials, such as seeds; soil, surfaces, materials, or rooms to be protected from fungal attack with a fungicidally effective amount of the active substances. The application can be carried out both before and after the infection of the plants, plant propagation materials, such as seeds; soil, surfaces, materials or rooms by the fungi.

An agrochemical composition comprises a fungicidally effective amount of a compound I. The term "fungicidally effective amount" denotes an amount of the composition or of the compounds I, which is sufficient for controlling harmful fungi on cultivated plants or in the protection of stored products or harvest or of materials and which does not result in a substantial damage to the treated plants, the treated stored products or harvest, or to the treated materials. Such an amount can vary in a broad range and is dependent on various factors, such as the fungal species to be controlled, the treated cultivated plant, stored product, harvest or material, the climatic conditions and the specific compound I used.

Plant propagation materials may be treated with compounds I as such or a composition comprising at least one compound I prophylactically either at or before planting or transplanting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials, such as seeds, e. g. by dusting, coating, or drenching, amounts of active substance of generally from 0.1 to 1000 g, preferably from 1 to 1000 g, more preferably from 1 to 100 g and most preferably from 5 to 100 g, per 100 kg of plant propagation material (preferably seeds) are required.

The user applies the agrochemical composition usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types (see also "Catalogue of pesticide formulation types and international coding system", Technical Monograph No. 2, 6th Ed. May 2008, CropLife International) are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials, such as seeds (e. g. GF). The compositions are prepared in a known manner, such as described by Mollet and Grubemann, Formulation technology, Wiley VCH, Weinheim, 2001; or by Knowles, New developments in crop protection product formulation, Agrow Reports DS243, T&F Informa, London, 2005. The invention also relates to agrochemical compositions comprising an auxiliary and at least one compound I. Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers, and binders.

Mixing the compounds I or the compositions comprising them in the use form as fungicides with other fungicides results in many cases in an expansion of the fungicidal spectrum of activity or in a prevention of fungicide resistance development. Furthermore, in many cases, synergistic effects are obtained (synergistic mixtures).

The following list of pesticides II, in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxy-strobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chloro-dincarb (A.1.20), famoxadone (A.1.21), fenamidone (A.1.21), methyl-N-[2-[(1,4-dimethyl-5-phenyl-pyrazol-3-yl)oxylmethyl]phenyl]-*N*-methoxy-carbamate (A.1.22), metyltetraprole (A.1.25), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-N,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);

   - inhibitors of complex III at Qᵢ site: cyazofamid (A.2.1), amisulbrom (A.2.2), [(6S,7R,8R)-8-benzyl-3-[(3-hydroxy-4-methoxy-pyridine-2-carbonyl)amino]-6-methyl-4,9-di-oxo-1,5-dioxonan-7-yl] 2-methylpropanoate (A.2.3), fenpicoxamid (A.2.4), florylpicoxamid (A.2.5), metarylpicoxamid (A.2.6);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (E)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)-pyridine-3-carboxamide (A.3.32), 2-(difluoromethyl)-*N*-[(3*R*)-1,1,3-trimethylindan-4-yl]-pyridine-3-carboxamide (A.3.33), 2-(difluoromethyl)-*N*-(3-ethyl-1,1-dimethyl-indan-4-yl)-pyridine-3-carboxamide (A.3.34), 2-(difluoromethyl)-*N*-[(3*R*)-3-ethyl-1,1-dimethyl-indan-4-yl]-pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]-pyridine-3-carboxamide (A.3.37), 2-(difluoromethyl)-*N*-(3-isobutyl-1,1-dimethyl-indan-4-yl)-pyridine-3-carboxamide (A.3.38), 2-(difluoromethyl)-*N*-[(3*R*)-3-isobutyl-1,1-dimethyl-indan-4-yl]pyridine-3-carboxamide (A.3.39) cyclobutrifluram (A.3.24);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromu-conazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothio-conazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(tri-fluoromethoxy)phenyl]-2-pyridyl]propan-2-ol (B.1.32), fluoxytioconazole (B.1.33), ipfen-trifluconazole (B.1.37), mefentrifluconazole (B.1.38), (2R)-2-[4-(4-chlorophenoxy)-2-(trifluoromethyl)phenyl]-1-(1,2,4-triazol-1-yl)propan-2-ol, (2S)-2-[4-(4-chlorophenoxy)-2-(trifluoromethyl)phenyl]-1-(1,2,4-triazol-1-yl)propan-2-ol, 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fena-rimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-diflu-oro-2-hydroxy-3-(1,2,4-triazol-1-yl)propyl]-3-pyridyl]oxy]benzonitrile (B.1.53), 2-[6-(4-bromo-phenoxy)-2-(trifluoromethyl)-3-pyridyl]-1-(1,2,4-triazol-1-yl)propan-2-ol (B.1.54), 2-[6-(4-chlo-rophenoxy)-2-(trifluoromethyl)-3-pyridyl]-1-(1,2,4-triazol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methyl-sulfanyl-acetamide (D.1.9), 2-[(3-ethynyl-8-methyl-6-quinolyl)oxy]-N-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-N-(2-fluoroethyl)-2-methylsulfanyl-acetamide (D.1.15), 4-(2-bromo-4-fluorophenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyr-azol-1-yl]acetyl]-4-piperidyl]-*N*-tetralin-1-yl-pyridine-2-carboxamide (G.5.6), 4-[1-[2-[5-cyclo-propyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1 -[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1H,5H-[1,4]di-thiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (!.2.4), fenoxanil (!.2.5);
J) Plant defence inducers
   - acibenzolar-S-methyl (J.1.1), probenazole (J.1.2), isotianil (J.1.3), tiadinil (J.1.4), prohexa-dione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flumetylsulforim (K.1.60), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxin-copper (K.1.22), proquinazid (K.1.23), seboctylamine (K.1.61), tebufloquin (K.1.24), tecloftalam (K.1.25), triazoxide (K.1.26), *N*'-(4-(4-chloro-3-trifluoromethyl-phenoxy)-2,5-dimethyl-phenyl)-*N*-ethyl-*N*-methyl formamidine (K.1.27), *N*'-(4-(4-fluoro-3-trifluoromethyl-phenoxy)-2,5-dimethyl-phenyl)-*N*-ethyl-*N*-methyl formamidine (K.1.28), *N*'-[4-[[3-[(4-chlorophenyl)methyl]-1,2,4-thiadiazol-5-yl]oxy]-2,5-dimethyl-phenyl]-*N-*ethyl-N-methyl-formamidine (K.1.29), *N'*-(5-bromo-6-indan-2-yloxy-2-methyl-3-pyridyl)-*N-*ethyl-N-methyl-formamidine (K.1.30), *N*'-[5-bromo-6-[1-(3,5-difluorophenyl)ethoxy]-2-methyl-3-pyridyl]-*N*-ethyl-*N*-methyl-formamidine (K.1.31), N'-[5-bromo-6-(4-isopropylcyclohexoxy)-2-methyl-3-pyridyl]-*N*-ethyl-*N*-methyl-formamidine (K.1.32), *N*'-[5-bromo-2-methyl-6-(1-phenylethoxy)-3-pyridyl]-*N*-ethyl-*N*-methyl-formamidine (K.1.33), *N*'-(2-methyl-5-trifluoromethyl-4-(3-trimethylsilanyl-propoxy)-phenyl)-N-ethyl-N-methyl formamidine (K.1.34), *N*'-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (Z)-3-amino-2-cyano-3-phenyl-prop-2-enoate (K.1.40), picarbutrazox (K.1.41), pentyl *N*-[6-[[(*Z*)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N*'-(2,5-dimethyl-4-phenoxy-phenyl)-*N*-ethyl-*N*-methyl-formamidine (K.1.53), aminopyrifen (K.1.54), fluopimomide (K.1.55), *N*'-[5-bromo-2-methyl-6-(1-methyl-2-propoxy-ethoxy)-3-pyridyl]-*N-*ethyl-N-methyl-formamidine (K.1.56), N'-[4-(4,5-dichlorothiazol-2-yl)oxy-2,5-dimethylphenyl]-*N*-ethyl-*N*-methyl-formamidine (K.1.57), flufenoxadiazam (K.1.58), N-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropanecarboxamide (WO2018/177894, WO 2020/212513);

In the binary mixtures the weight ratio of the component 1) and the component 2) generally depends from the properties of the components used, usually it is in the range of from 1:10,000 to 10,000:1, often from 1:100 to 100:1, regularly from 1:50 to 50:1, preferably from 1:20 to 20:1, more preferably from 1:10 to 10:1, even more preferably from 1:4 to 4:1 and in particular from 1:2 to 2:1. According to further embodiments, the weight ratio of the component 1) and the component 2) usually is in the range of from 1000:1 to 1:1, often from 100: 1 to 1:1, regularly from 50:1 to 1:1, preferably from 20:1 to 1:1, more preferably from 10:1 to 1:1, even more preferably from 4:1 to 1:1 and in particular from 2:1 to 1:1. According to further embodiments, the weight ratio of the component 1) and the component 2) usually is in the range of from 20,000:1 to 1:10, often from 10,000:1 to 1:1, regularly from 5,000:1 to 5:1, preferably from 5,000:1 to 10:1, more preferably from 2,000:1 to 30:1, even more preferably from 2,000:1 to 100:1 and in particular from 1,000:1 to 100:1. According to further embodiments, the weight ratio of the component 1) and the component 2) usually is in the range of from 1:1 to 1:1000, often from 1:1 to 1:100, regularly from 1:1 to 1:50, preferably from 1:1 to 1:20, more preferably from 1:1 to 1:10, even more preferably from 1:1 to 1:4 and in particular from 1:1 to 1:2. According to further embodiments, the weight ratio of the component 1) and the component 2) usually is in the range of from 10:1 to 1:20,000, often from 1:1 to 1:10,000, regularly from 1:5 to 1:5,000, preferably from 1:10 to 1:5,000, more preferably from 1:30 to 1:2,000, even more preferably from 1:100 to 1:2,000 to and in particular from 1:100 to 1:1,000.

In the ternary mixtures, i.e. compositions comprising the component 1) and component 2) and a compound III (component 3), the weight ratio of component 1) and component 2) depends from the properties of the active substances used, usually it is in the range of from 1:100 to 100:1, regularly from 1:50 to 50:1, preferably from 1:20 to 20:1, more preferably from 1:10 to 10:1 and in particular from 1:4 to 4:1, and the weight ratio of component 1) and component 3) usually it is in the range of from 1:100 to 100:1, regularly from 1:50 to 50:1, preferably from 1:20 to 20:1, more preferably from 1:10 to 10:1 and in particular from 1:4 to 4:1. Any further active components are, if desired, added in a ratio of from 20:1 to 1:20 to the component 1). These ratios are also suitable for mixtures applied by seed treatment.

Preference is given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qᵢ site in group A), more preferably selected from compounds (A.2.1), (A.2.3), (A.2.4) and (A.2.6); particularly selected from (A.2.3), (A.2.4) and (A.2.6).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.24), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.24), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration inhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.33), (B.1.34), (B.1.37), (B.1.38), (B.1.43), (B.1.46), (B.1.53), (B.1.54) and (B.1.55); particularly selected from (B.1.5), (B.1.8), (B.1.10), (B.1.17), (B.1.22), (B.1.23), (B.1.25), (B.1.33), (B.1.34), (B.1.37), (B.1.38), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6), (C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.3), (G.5.4), (G.5.5), G.5.6), G.5.7), (G.5.8), (G.5.9), (G.5.10) and (G.5.11); particularly selected from (G.3.1), (G.5.1) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (!.2.2) and (!.2.5).

Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.47), (K.1.57), (K.1.58) and (K.1.59); particularly selected from (K.1.41), (K.1.44), (K.1.47), (K.1.57), (K.1.58) and (K.1.59).

The compositions comprising mixtures of active ingredients can be prepared by usual means, e. g. by the means given for the compositions of compounds I.

### Examples:

### Synthetic process

### Example 1: Methyl (2E)-2-methoxyimino-2-[2-[[(E)-(1-methyl-3-phenyl-prop-2-ynylidene)amino]-oxymethyl]phenyl]acetate (examples numbered according to Table S below)

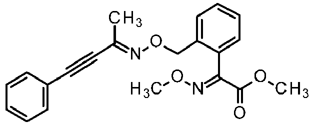

### Step 1: 4-Phenylbut-3-yn-2-one

To a stirred solution of phenyl acetylene (10 g, 1 eq.) in THF (150 ml) at -78 °C was added n-butyl lithium (73 ml, 1.6 M solution in hexane 1.2 eq.) dropwise and stirred at the same temperature for 30 minutes. Then ethyl acetate (12 ml, 1.2 eq.) in THF (15ml) was added dropwise at -78 °C followed by boron trifluoride diethyl ether complex (33 ml, 50% solution, 1.2 eq.) at the same temperature and the reaction mixture was stirred for 30 min. After completion of the reaction indicated by TLC, the reaction mixture was quenched with saturated aqueous ammonium chloride solution (200 ml) and then water (100 mL) was added and the mixture was extracted with ethyl acetate (3x 200 mL) and the combined organic phase was washed using brine and dried over sodium sulfate. The solvent was removed to obtain crude product, which was purified by combi flash column chromatography using 15-20% ethyl acetate in heptane as mobile phase to obtain 4-phenylbut-3-yn-2-one (10 g, 71% yield). ¹H NMR (500 MHz, DMSO-d6): δ 7.67-7.65(m, 2H), 7.59 - 7.56 (m, 1H), 7.37 - 7.15 (m, 2H), 2.45 (s, 3H).

### Step 2: 4-Phenylbut-3-yn-2-one oxime

To a stirred solution of 4-phenylbut-3-yn-2-one (200 mg, 1 eq.) in methanol (10 ml) and water (2.0 ml) at 25 °C hydroxylamine hydrochloride (190 mg, 2 eq.) and sodium acetate (394 mg, 2 eq.) were added. The mixture was stirred at about 20 °C for 4 h. After TLC indicated completion of the reaction, the mixture was evaporated to dryness and water (30 ml) added to it and extracted with ethyl acetate (3x 30 ml) and the combined organic phase was washed using brine and dried over sodium sulfate. The solvent was removed to obtain 220 mg crude 4-phenylbut-3-yn-2-one oxime as a mixture of both cis and trans (50:50) isomers, which was directly used for the next step without any purification.

### Step 3: Methyl (2E)-2-methoxyimino-2-[2-[[(E)-(1-methyl-3-phenyl-prop-2-ynylidene)amino]oxymethyl]phenyl]acetate

To a stirred solution of 4-phenylbut-3-yn-2-one oxime (15 g as a mixture of cis:trans isomers) in AcN (200 ml) at 10 °C, methyl (2E)-2-[2-(chloromethyl)phenyl]-2-methoxyimino-acetate (82 ml, 33% solution in DMF) was added followed by cesium carbonate (61.4 g, 2 eq.). The reaction mixture was then stirred at 25 °C for 16 h. After TLC indicated completion of the reaction, water (200 ml) was added and extracted with ethyl acetate (3x 200 ml). The combined organic phase was washed with brine and dried over sodium sulfate. The solvent was removed to obtain crude product, which was purified by column chromatography to obtain 7.5 g (21% yield) of pure title compound. ¹H NMR (500 MHz, DMSO-d6): δ 7.54-7.52 (m, 2H), 7.46-7.41 (m, 6H), 7.24-7.22 (m, 1H), 5.01 (s, 2H), 3.96 (s, 3H), 3.76 (s, 3H), 1.99 (s, 3H).

Alternatively, the title compound was obtained as follows.

### Step 2a: Methyl (2E)-2-[2-(aminooxymethyl)phenyl]-2-methoxyimino-acetate

To a stirred solution of methyl (2E)-2-[2-[(1,3-dioxoisoindolin-2-yl)oxymethyl]phenyl]-2-meth-oxyimino-acetate (400 mg, 1 eq.) in methanol (10 ml) at 25 °C, hydrazine hydrate (54 mg, 1 eq.) was added and stirred for 30 minutes at the same temperature. After TLC showed completion of the reaction, the solvent was evaporated and the crude reaction mixture was subjected to column chromatography to afford the pure product (150 mg).¹H NMR (300 MHz, DMSO-d6): δ 7.41-7.33 (m, 3H), 7.18-7.16 (m, 1H), 5.93 (s, 2H), 4.39 (s, 2H), 3.92 (s, 3H), 3.75 (s, 3H).

### Step 3a: Methyl (2E)-2-methoxyimino-2-[2-[[(E)-(1-methyl-3-phenyl-prop-2-ynylidene)amino]oxymethyl]phenyl]acetate

To a stirred solution of 4-phenylbut-3-yn-2-one (240 mg, 1.2 eq.) and methyl (2E)-2-[2-(ami-nooxymethyl)phenyl]-2-methoxyimino-acetate (330 mg, 1 eq.) in methanol (5 ml), acetic acid (0.2 ml) was added and heated at 65 °C and stirred at this temperature for 2 h. After TLC indicated completion of the reaction, the mixture was evaporated to dryness and water (50 ml) added to it and extracted with ethyl acetate (3x 30 ml) and the combined organic phase was washed using brine and dried over sodium sulfate. The crude product was purified by column chromatography using 20% ethyl acetate in heptane as mobile phase to obtain 160 mg (32 % yield) of the title compound.

### Example 2: (2E)-2-Methoxyimino-N-methyl-2-[2-[[(E)-(1-methyl-3-phenyl-prop-2-ynylidene)-amino]oxymethyl]phenyl]acetamide

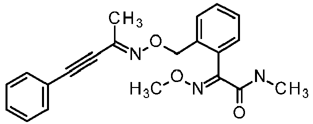

To a stirred solution of methyl (2E)-2-methoxyimino-2-[2-[[(E)-(1-methyl-3-phenyl-prop-2-yn-ylidene)amino]oxymethyl]phenyl]acetate (Example 1) (120 mg, 1 eq.) in THF (5 mL) at 25 °C was added methyl amine (0.4 mL, 40% solution in water) and was stirred for 4 h. After completion of the reaction as indicated by TLC, the reaction mixture was evaporated and washed with pentane (5 mL × 3 times) and was purified by combi flash column chromatography to obtain 11 mg (9% yield) of pure title compound.

### Example 15: Methyl (2E)-2-methoxyimino-2-[3-methyl-2-[[(E)-(1-methyl-3-phenyl-prop-2-ynyl-idene)amino]oxymethyl]phenyl]acetate

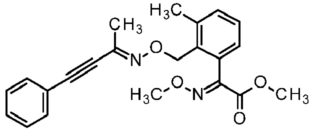

To a stirred solution of 4-phenylbut-3-yn-2-one oxime (1 g, as a mixture of cis:trans isomers, 1 eq.) in acetonitrile (15 ml), cesium carbonate (4.08 g, 2 eq.) and methyl (2E)-2-[2-(bromomethyl)-3-methyl-phenyl]-2-methoxyimino-acetate (1.7 g, 1 eq.) dissolved in acetonitrile (5 ml) were added at 25 °C and stirred at this temperature for 4 h. After TLC indicated completion of the reaction, the mixture was filtered through celite and washed with ethyl acetate (3x 30 ml). The filtrate was then evaporated and purified by column chromatography using 15% ethyl acetate in heptane as mobile phase to obtain 1.1 g (yield 51%) of the title compound.

¹H NMR (500 MHz, DMSO-d6): δ 7.54-7.52 (m, 2H), 7.47-7.42 (m, 3H), 7.34-7.29 (m, 2H), 7.04-7.02 (m, 1H), 5.01 (bs, 2H), 3.93 (s, 3H), 3.74 (s, 3H) 2.40 (s, 3H), 1.96 (s, 3H).

### Example 16: (2E)-2-Methoxyimino-N-methyl-2-[3-methyl-2-[[(E)-(1-methyl-3-phenyl-prop-2-ynyl-idene)amino]oxymethyl]phenyl]acetamide

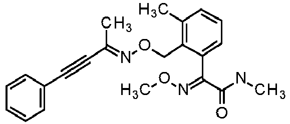

To a stirred solution of methyl (2E)-2-methoxyimino-2-[2-[[(E)-(1-methyl-3-phenyl-prop-2-yn-ylidene)amino]oxymethyl]phenyl]acetate (Example 15) (750 mg, 1 eq.) in THF (10 ml) at 25 °C, methyl amine (0.6 ml, 40% solution in water) was added and stirred for 5 h. After completion of the reaction as indicated TLC, water (50 ml) was added to the reaction mixture and was extracted by using ethyl acetate (3x 30 ml) and the combined organic phase was washed using brine and dried over sodium sulfate. The solvent was removed to obtain crude product, which was purified by column chromatography using 35% ethyl acetate in heptane as mobile phase to obtain 450 mg (60% yield) of pure title compound. ¹H NMR (500 MHz, DMSO-d6): δ 8.21 (s, 1H) 7.54-7.52 (m, 2H), 7.46-7.42 (m, 3H), 7.31-7.26 (m, 2H), 6.96-6.94 (m, 1H), 5.00 (bs, 2H), 3.88 (s, 3H), 2.70 (s, 3H) 2.39 (s, 3H), 1.96 (s, 3H).

### Example 12: methyl(2E)-2-methoxyimino-2-[2-[[(E)-[1-methyl-3-[4-(trifluoromethoxy)phenyl]prop-2-ynylidene]amino]oxymethyl]phenyl]acetate

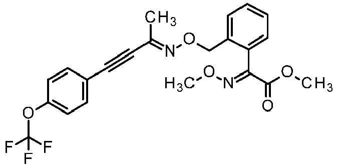

To a stirred solution of 4-[4-(trifluoromethoxy)phenyl]but-3-yn-2-one oxime (300 mg as a mixture of cis:trans isomers, 1 eq.) in acetonitrile (8 ml), cesium carbonate (820 mg, 2 eq.) and methyl (2E)-2-[2-(bromomethyl)phenyl]-2-methoxyimino-acetate (350 mg, 1 eq.) were added at 25 °C and stirred at this temperature for 2 h. After TLC indicated completion of the reaction, the mixture was filtered through Celite and washed with ethyl acetate (25 ml). The filtrate was then evaporated and purified by column chromatography using 15-20% ethyl acetate in heptane as mobile phase to obtain the title compound (500 mg, 90% yield). ¹H NMR (500 MHz, DMSO-d6): δ 7.69-7.67 (m, 2H), 7.46 - 7.24 (m, 5H), 7.24-7.22 (m, 1H), 5.01 (s, 2H), 3.95 (s, 3H), 3.76 (s, 3H), 1.99 (s, 3H).

### Example 11: (2E)-2-Methoxyimino-N-methyl-2-[2-[[(E)-[1-methyl-3-[4-(trifluoromethoxy)phenyl]-prop-2-ynylidene]amino]oxymethyl]phenyl]acetamide

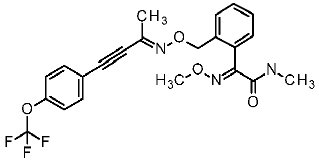

To a stirred solution of methyl (2E)-2-methoxyimino-2-[2-[[(E)-[1-methyl-3-[4-(trifluorometh-oxy)phenyl]prop-2-ynylidene]amino]oxymethyl]phenyl]acetate (Example 12 above) in THF (6 ml) at room temperature, methyl amine (0.6 ml, 40% solution in water) was added and stirred for 2 h. After completion of the reaction as indicated TLC, water (15 ml) was added to the reaction mixture and was extracted by using ethyl acetate (3x 15ml) and the combined organic phase was washed using brine and dried over sodium sulfate. The solvent was removed to obtain crude product, which was purified by column chromatography using 30-35% ethyl acetate in heptane as mobile phase to obtain the title compound (220mg, 73% yield). ¹H NMR (500 MHz, DMSO-d6): δ 8.26 (s, 1H), 8.25 - 8.25 (d, J = 5Hz, 2H), 7.69 - 7.67 (m, 5H), 7.36-7.15 (m, 1H), 5.02 (s, 2H), 3.90 (s, 3H), 2.71 (d, J = 5Hz, 3H), 2.02 (s, 3H).

### Example 22: methyl (2E)-2-methoxyimino-2-[3-methyl-2-[[(E)-[1-methyl-3-[4-(trifluoromethoxy)-phenyl]prop-2-ynylidene]amino]oxymethyl]phenyl]acetate

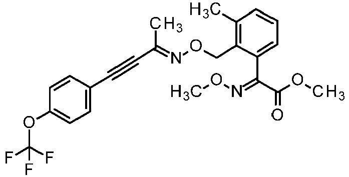

### Step 1: Trimethyl-[2-[4-(trifluoromethoxy)phenyl]ethynyl]silane

To a stirred solution of 1-iodo-4-(trifluoromethoxy)benzene (500 mg 1 eq.) in THF (5 ml) under nitrogen, triethylamine (3.5 ml, 3 eq.), trimethylsilyl acetylene (0.731 ml, 3 eq.), Cul (33 mg, 0.1 eq.) and PdCl₂(PPh₃)₂ (122 mg, 0.1 eq.) were added and stirred at 60 °C for 2 h. After TLC indicated completion of the reaction, the mixture was filtered through Celite and water (10 ml) was added. The organic phase was extracted by using ethyl acetate (3x 15 ml) and the combined organic phase was washed using brine and dried over sodium sulfate. The solvent was removed to obtain crude product, which was purified by column chromatography to obtain trimethyl-[2-[4-(trifluoromethoxy)phenyl]ethynyl]silane (400 mg, 89% yield). ¹H NMR (500 MHz, CDCl₃): δ 7.29 (d, J = 8.00 Hz, 2H), 6.95 (d, J = 8.00 Hz, 2H), 0.06 (s, 9H).

### Step 2: 1-Ethynyl-4-(trifluoromethoxy)benzene

To a stirred solution of trimethyl-[2-[4-(trifluoromethoxy)phenyl]ethynyl]silane (4.4 g, 1 eq.) in THF (44 ml), tetrabutylammonium fluoride (TBAF) (1.7 ml, 1 M solution in THF) was added dropwise at 0 °C. The resulting solution was stirred at 0 to 5 °C for 5 min. After completion of the reaction as indicated by TLC, the reaction mixture was quenched by adding water (50 ml) at 0 °C. The organic phase was extracted by using ethyl acetate (3x 25 ml) and the combined organic phase was washed using brine and dried over sodium sulfate. The solvent was removed to obtain crude product, which was purified by column chromatography using 2-5% ethyl acetate in heptane as mobile phase to obtain 1-ethynyl-4-(trifluoromethoxy)benzene (1 g, 41% yield). ¹H NMR (500 MHz, CDCl₃): δ 7.43 (d, J = 8.00 Hz, 2H), 7.08 (d, J = 8.00 Hz, 2H), 3.01 (s, 1H).

### Step 3: 4-[4-(Trifluoromethoxy)phenyl]but-3-yn-2-one

To a stirred solution of 1-ethynyl-4-(trifluoromethoxy)benzene (1 g, 1 eq.) in THF (10 ml) at -78 °C, 2.5 M solution of n-butyl lithium (2.58 ml, 2.5 M in hexane, 1.2 eq.) was added dropwise and stirred at the same temperature for 20 min. Ethyl acetate (0.63 ml, 1.2 eq.) in THF (2 ml) was then added dropwise at -78 °C followed by boron trifluoride diethyl ether complex (0.72 ml, 1.2 eq.) at the same temperature and stirred the reaction mixture for 1 h. After completion of the reaction as indicated by TLC, the reaction mixture was quenched with saturated ammonium chloride solution and extracted with ethyl acetate (3x 15 ml) and the combined organic phase was washed using brine and dried over sodium sulfate. After removal of the solvent, the crude product was purified by column chromatography using 15-20% ethyl acetate in heptane as mobile phase to obtain 4-[4-(trifluoromethoxy)phenyl]but-3-yn-2-one (0.5 g, 41 % yield). ¹H NMR (500 MHz, CDCl₃): δ 7.64 (d, J = 8.00 Hz, 2H), 7.26 (d, J = 8.00 Hz, 2H), 3.1 (s, 3H).

### Step 4: 4-[4-(Trifluoromethoxy)phenyl]but-3-yn-2-one oxime

To a stirred solution of 4-[4-(trifluoromethoxy)phenyl]but-3-yn-2-one (500 mg, 1 eq.) in a mixture of methanol: water (4:1) at 10 °C, hydroxylamine hydrochloride (302 mg, 2 eq.) and sodium acetate (359 mg, 2 eq.) were added. The mixture was stirred at 10 to 15 °C for 2 h. After TLC indicated completion of the reaction, the mixture was evaporated to dryness and water (15 ml) was added and extracted with ethyl acetate (3x 15 ml). The combined organic phase was washed using brine and dried over sodium sulfate and solvent was removed to obtain 4-[4-(tri-fluoromethoxy)phenyl]but-3-yn-2-one oxime (500 mg, 93% yield) as a mixture of E and Z isomers (50:50), which was directly used for the next step without any purification.

### Step 5: Methyl (2E)-2-methoxyimino-2-[3-methyl-2-[[(E)-[1-methyl-3-[4-(trifluoromethoxy)phenyl]prop-2-ynylidene]amino]oxymethyl]phenyl]acetate

To a stirred solution of 4-[4-(trifluoromethoxy)phenyl]but-3-yn-2-one oxime (250 mg as a mixture of cis:trans isomers, 1 eq.) in acetonitrile (8 ml), cesium carbonate (670 mg, 2 eq.) and methyl (2E)-2-[2-(bromomethyl)-3-methyl-phenyl]-2-methoxyimino-acetate (308 mg, 1 eq.) were added at 25 °C and stirred at this temperature for 2 h. After TLC indicated completion of the reaction, the mixture was filtered through Celite and washed with ethyl acetate (25 ml). The filtrate was then evaporated and purified by column chromatography using 15-20% ethyl acetate in heptane as mobile phase to obtain the title compound (400 mg, 84% yield). ¹H NMR (500 MHz, DMSO-d6): δ 7.77 (d, J = 8 Hz, 2H), 7.49 (d, J = 8 Hz, 2H), 7.34 - 7.29 (m, 2H), 7.01 - 7.00 (m, 1H), 5.50 (bs, 1H), 5.00 (bs, 1H), 3.86 (s, 3H), 3.65 (s, 3H), 2.46 (s, 3H), 2.05 (s, 3H).

### Example 21: (2E)-2-Methoxyimino-N-methyl-2-[3-methyl-2-[[(E)-[1-methyl-3-[4-(trifluorometh-oxy)phenyl]prop-2-ynylidene]amino]oxymethyl]phenyl]acetamide

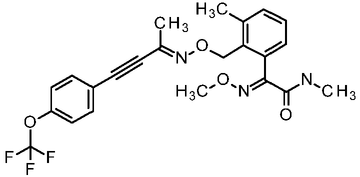

To a stirred solution of methyl (2E)-2-methoxyimino-2-[3-methyl-2-[[(E)-[1-methyl-3-[4-(tri-fluoromethoxy)phenyl]prop-2-ynylidene]amino]oxymethyl]phenyl]acetate (Example 22 above) (300 mg, 1 eq) in THF (6 ml) at 25 °C, methyl amine (0.6 ml, 40% solution in water) was added and stirred for 2 h. After completion of the reaction as indicated TLC, water (15 ml) was added to the reaction mixture and was extracted by using ethyl acetate (3x 15ml) and the combined organic phase was washed using brine and dried over sodium sulfate. After removal of the solvent, the crude product was purified by column chromatography using 30-35% ethyl acetate in heptane as mobile phase to obtain the title compound (220 mg 71% yield). ¹H NMR (500 MHz, DMSO-d6): δ 8.2 (bs, 1H), 7.67 (d, J = 5 Hz, 2H), 7.44 (d, J = 10 Hz, 2H), 7.30 - 7.27 (m, 2H), 6.9 - 7.00 (m, 1H), 5.00 (bs, 2H), 3.3 (s, 3H), 2.68 (d, 3H), 2.39 (s, 3H), 1.97 (s, 3H).

The following examples in Table S were synthesized as described above and characterized by LCMS as described in Table L.

**Table L: LCMS Methods**

| **Method A** | **Device details** |
|---|---|
| Column: Agilent Eclipse Plus C18 | LCMS2020 (Shimadzu) |
| (50 mm × 4.6 mm × 3 µm) | Ionization source: ESI |
| Mobile Phase: | Mass range: 100 - 800 amu |
| A: 10 mM Ammonium formate in water. | Polarity: Dual; Mode: Scan |
| B: 0.1 % Formic acid in acetonitrile. | LC System: Nexera High pressure gradient system, Binary pump |
| Gradient: 10 % B to 100 % B in 1.5 min. Hold 1 min 100% B. 1 min 10 % B. Run time: 3.50 or 3.75 min. | |
| | Detector: PDA |
| Flow: 1.2 ml/min; Column oven: 30°C/40°C | Scan wavelength: 220 nm / max plot |

| **Method B** | **Device details** |
|---|---|
| Column: Xbridge Shield RP18 (2.1 × 50mm × 5 µm) | Agilent 1200 & 6120B |
| Mobile Phase: | Ionization source: ESI |
| A: H₂O+10mM NH₄HCO₃; B: Acetonitrile | Mass range: 100 - 1000 amu |
| Gradient: 5% B in 0.40 min and 5-95% B at 0.40-3.40 min, hold on 95% B for 0.45min, 95-5% B in 0.01 min, Run time: 4.50 min. | Polarity: positive; Mode: Scan |
| | LC System: Nexera High pressure gradient system, Binary pump |
| Flow: 0.8 ml/min; Column oven: 40°C | Detector: PDA |
| | Scan wavelength: 220 nm |

| **Method C** | **Device details** |
|---|---|
| Column: Xbridge Shield RP18 (2.1 × 50mm × 5 µm) | Agilent 1200 & 6120B |
| Mobile Phase: | Ionization source: ESI |
| A: H₂O+10mM NH₄HCO₃; B: Acetonitrile. | Mass range: 100 - 1000 amu |
| Gradient: 50%B in 0.40min; 50-100% B at 0.40-3.40 min, hold on 100% B for 0.45 min, 100-50% B in 0.01 min. Run time: 4.50 min. | Polarity: positive; Mode: Scan |
| | LC System: Nexera High pressure gradient system, Binary pump |
| Flow: 0.8 ml/min; Column oven: 40°C | Detector: PDA |
| | Scan wavelength: 220 nm |

| **Method D** | **Device details** |
|---|---|
| Column: Xbridge Shield RP18 (2.1 × 50mm × 5 µm) | Agilent 1200 & 6120B |
| Mobile Phase: | Ionization source: ESI |
| A: H₂O+10mM NH₄HCO₃; B: Acetonitrile. | Mass range: 100 - 1000 amu |
| Gradient: 50%B in 0.40min; 50-100% B at 0.40-3.40 min, hold on 100% B for 0.45 min, 100-50% B in 0.01 min. Run time: 4.50 min. | Polarity: positive; Mode: Scan |
| | LC System: Nexera High pressure gradient system, Binary pump |
| Flow: 0.8 ml/min; Column oven: 40°C | Detector: PDA |
| | Scan wavelength: 220 nm |

| **Method E** | **Device details** |
|---|---|
| Column: Kinetex -C18 (50 mm × 2.1 mm × 5 µm) | Shimadzu LC-20AD&MS 2020 |
| Mobile Phase: | Ionization source: ESI |
| A: 0.037% Trifluoroacetic acid in water. | Mass range: 100 - 1000 amu |
| B: 0.018% Trifluoroacetic acid in acetonitrile. | Polarity: Positive; Mode: Scan |
| Gradient: 5% B in 0.40 min; 5-95% B at 0.4-3.0 min, hold on 95% B for 1.00min; 95-5%B in | LC System: Nexera High pressure gradient system, Binary pump |
| 0.01 min. | Detector: DAD |
| Flow: 1.0 mL/min; Column oven: 40°C | Scan wavelength: 220 nm / max plot |

| **Method F** | **Device details** |
|---|---|
| Column: Agilent Eclipse Plus C18 (50 mm × 4.6 mm × 3 µm) | LCMS2020 (Shimadzu) |
| | Ionization source: ESI |
| Mobile Phase: | Mass range: 100 - 800 amu |
| A: 10 mM Ammonium formate in water. | Polarity: Dual; Mode: Scan |
| B: Acetonitrile | LC System: Nexera High pressure gradient system, Binary pump |
| Gradient: 10 % B to 100 % B in 5 min. Hold 3 min 100 % B. 2 min 10 % B. Run time: 10 min. | |
| | Detector: PDA |
| Flow: 1.2 ml/min; Column oven: 40°C | Scan wavelength: 220 nm / max plot |

**Table S:**

| **No.** | **Structure** | **Rₜ [min]** | **Mass** | **Method** |
|---|---|---|---|---|
| 1 | 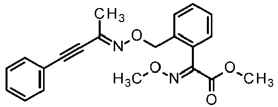 | 2.04 | 365 | A |
| 2 | 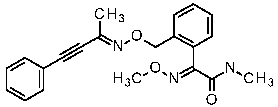 | 1.93 | 364 | A |
| 3 | 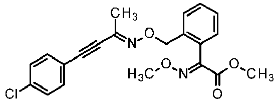 | 2.21 | 399 | A |
| 4 | 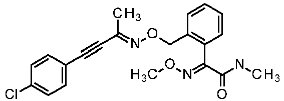 | 2.1 | 398 | A |
| 5 | 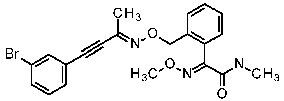 | 2.15 | 444 | A |
| 6 | 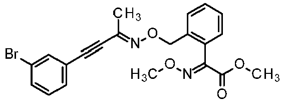 | 2.07 | 443 | A |
| 7 | 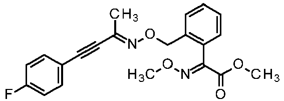 | 2.11 | 383 | A |
| 8 | 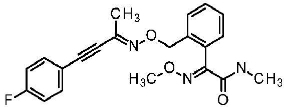 | 2.04 | 382 | A |
| 9 | 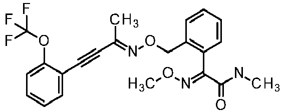 | 2.10 | 448 | A |
| 10 | 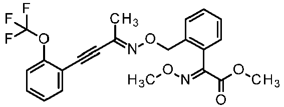 | 2.23 | 449 | A |
| 11 | 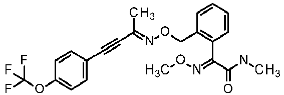 | 2.14 | 448 | A |
| 12 | 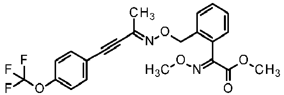 | 2.23 | 449 | A |
| 13 | 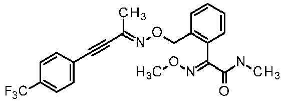 | 2.13 | 432 | A |
| 14 | 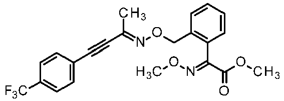 | 2.21 | 433 | A |
| 15 | 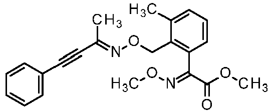 | 2.17 | 379 | A |
| 16 | 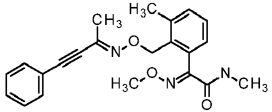 | 2.05 | 378 | A |
| 17 | 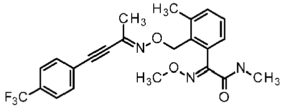 | 2.18 | 446 | A |
| 18 | 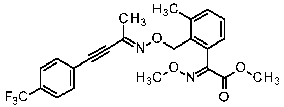 | 2.26 | 447 | A |
| 19 | 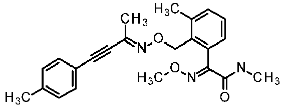 | 2.16 | 392 | A |
| 20 | 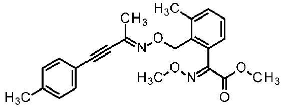 | 2.27 | 383 | A |
| 21 | 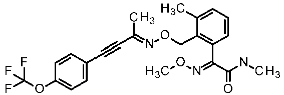 | 2.19 | 462 | A |
| 22 | 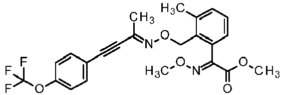 | 2.08 | 463 | A |
| 23 | 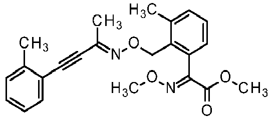 | 2.24 | 393 | A |
| 24 | 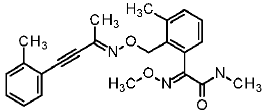 | 2.14 | 392 | A |
| 25 | 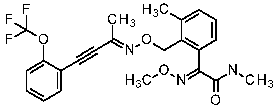 | 2.17 | 462 | A |
| 26 | 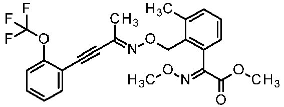 | 2.25 | 463 | A |
| 27 | 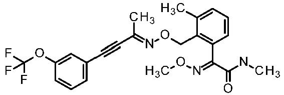 | 2.19 | 462 | A |
| 28 | 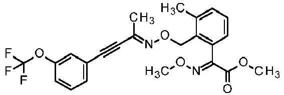 | 2.28 | 463 | A |
| 29 | 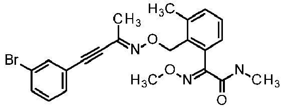 | 2.19 | 456 | A |
| 30 | 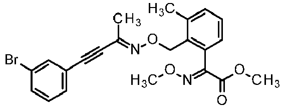 | 2.3 | 459 | A |
| 31 | 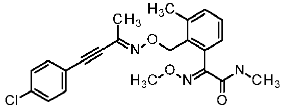 | 2.16 | 412 | A |
| 32 | 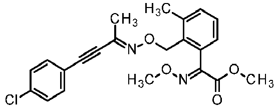 | 2.2 | 413 | A |
| 33 | 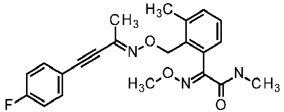 | 2.07 | 396 | A |
| 34 | 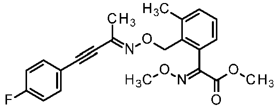 | 2.18 | 397 | A |
| 35 | 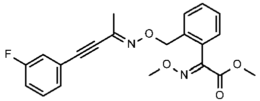 | 2.07 | 383 | A |
| 36 | 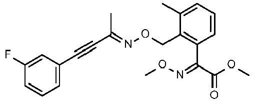 | 2.18 | 397 | A |
| 37 | 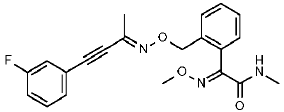 | 2.01 | 382 | A |
| 38 | 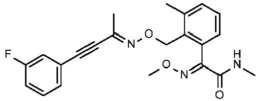 | 2.06 | 396 | A |
| 39 | 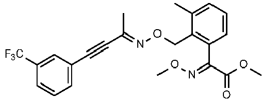 | 2.26 | 447 | A |
| 40 | 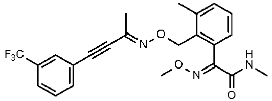 | 2.16 | 446 | A |
| 41 | 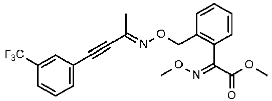 | 2.2 | 433 | A |
| 42 | 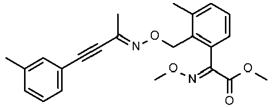 | 2.24 | 393 | A |
| 43 | 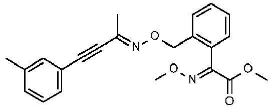 | 2.19 | 379 | A |
| 44 | 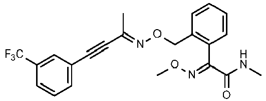 | 2.10 | 432 | A |
| 45 | 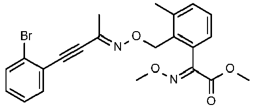 | 2.25 | 459 | A |
| 46 | 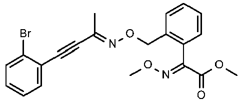 | 2.2 | 445 | A |
| 47 | 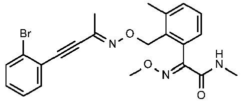 | 2.15 | 458 | A |
| 48 | 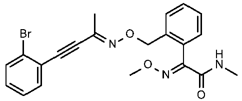 | 2.14 | 444 | A |
| 49 | 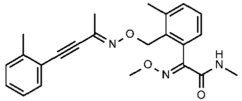 | 2.14 | 392 | A |
| 50 | 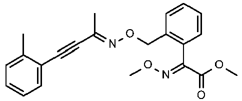 | 2.21 | 379 | A |
| 51 | 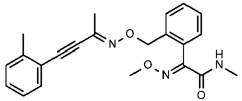 | 2.09 | 378 | A |
| 52 | 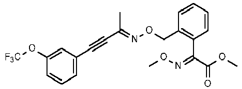 | 2.24 | 449 | A |
| 53 | 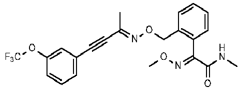 | 2.14 | 448 | A |
| 54 | 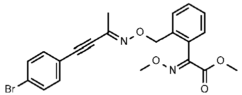 | 2.12 | 445 | A |
| 55 | 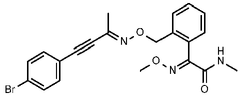 | 2.14 | 444 | A |
| 56 | 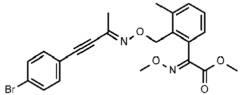 | 2.29 | 459 | A |
| 57 | 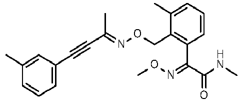 | 2.16 | 392 | A |
| 58 | 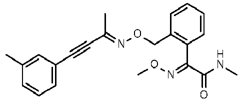 | 2.09 | 378 | A |
| 59 | 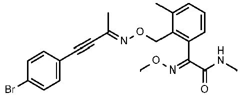 | 2.19 | 456 | A |
| 60 | 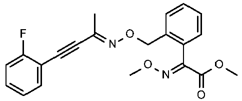 | 2.1 | 383 | A |
| 61 | 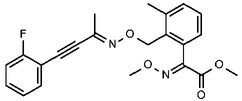 | 2.15 | 397 | A |
| 62 | 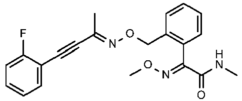 | 1.99 | 382 | A |
| 63 | 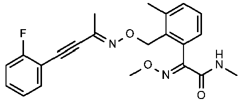 | 2.04 | 396 | A |
| 64 | 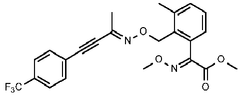 | 2.26 | 447 | A |
| 65 | 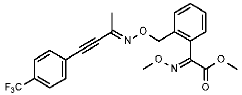 | 2.21 | 433 | A |
| 66 | 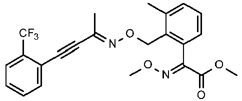 | 2.23 | 447 | A |
| 67 | 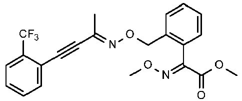 | 2.19 | 433 | A |
| 68 | 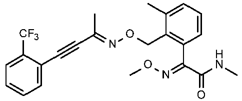 | 2.12 | 446 | A |
| 69 | 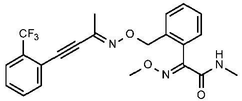 | 2.09 | 432 | A |
| 70 | 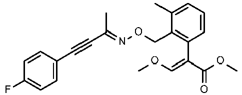 | 2.17 | 396 | A |
| 71 | 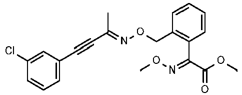 | 2.09 | 399 | A |
| 72 | 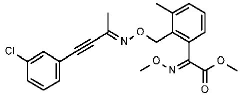 | 2.2 | 413 | A |
| 73 | 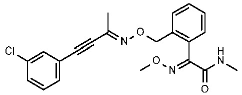 | 2.1 | 398 | A |
| 74 | 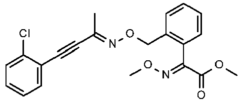 | 2.18 | 399 | A |
| 75 | 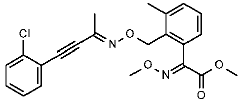 | 2.2 | 413 | A |
| 76 | 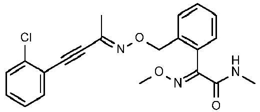 | 2.08 | 398 | A |
| 77 | 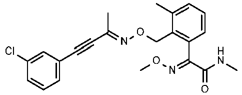 | 2.16 | 412 | A |
| 78 | 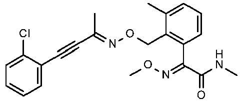 | 4.6 | 412 | F |
| 79 | 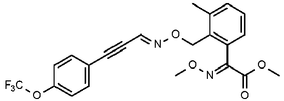 | 2.22 | 449 | A |
| 80 | 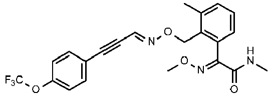 | 2.14 | 448 | A |
| 81 | 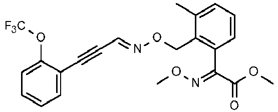 | 2.21 | 449 | A |
| 82 | 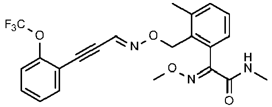 | 2.06 | 448 | A |
| 83 | 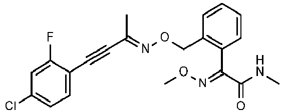 | 2.02 | 416 | A |
| 84 | 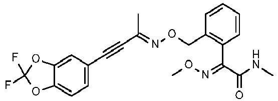 | 1.84 | 444.1 | C |
| 85 | 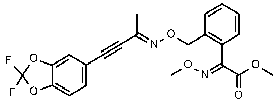 | 2.26 | 445.1 | C |
| 86 | 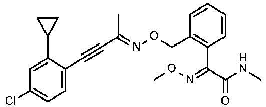 | 2.25 | 438.2 | C |
| 87 | 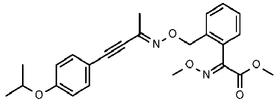 | 2.29 | 423.2 | C |
| 88 | 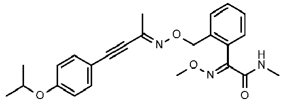 | 1.88 | 422.2 | C |
| 89 | 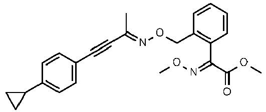 | 2.31 | 405.2 | C |
| 90 | 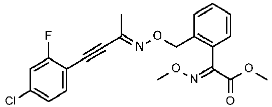 | 2.19 | 417.1 | C |
| 91 | 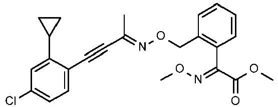 | 2.62 | 439.1 | C |
| 92 | 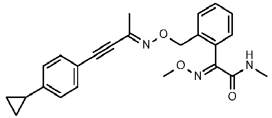 | 3.43 | 404.2 | B |
| 93 | 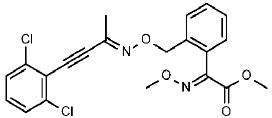 | 2.20 | 433.1 | C |
| 94 | 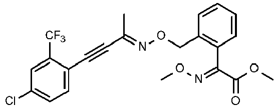 | 2.49 | 467.1 | C |
| 95 | 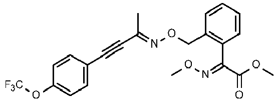 | 2.42 | 437.2 | C |
| 96 | 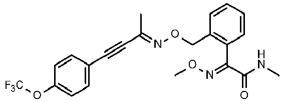 | 2.02 | 436.2 | C |
| 97 | 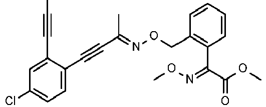 | 2.54 | 437.1 | C |
| 98 | 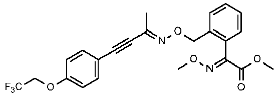 | 2.14 | 463.1 | C |
| 99 | 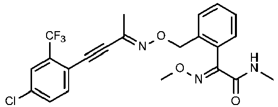 | 2.13 | 466.1 | C |
| 100 | 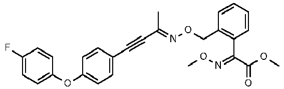 | 2.52 | 475.1 | C |
| 101 | 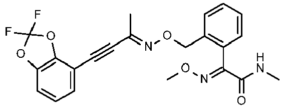 | 1.75 | 444.1 | C |
| 102 | 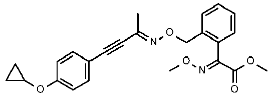 | 2.17 | 421.2 | C |
| 103 | 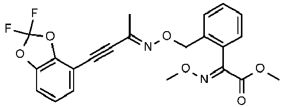 | 2.19 | 445.1 | C |
| 104 | 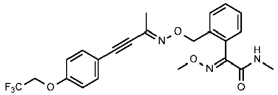 | 1.72 | 462.2 | C |
| 105 | 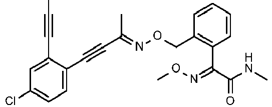 | 2.17 | 436.1 | C |
| 106 | 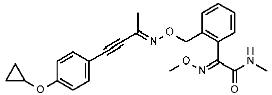 | 1.73 | 420.2 | C |
| 107 | 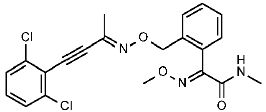 | 1.76 | 432.1 | C |
| 108 | 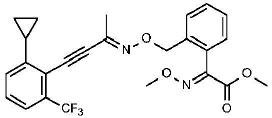 | 2.48 | 473.2 | C |
| 109 | 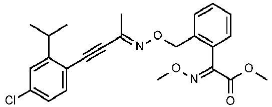 | 2.79 | 441.2 | C |
| 110 | 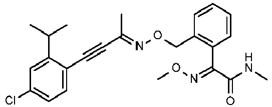 | 2.45 | 440.2 | C |
| 111 | 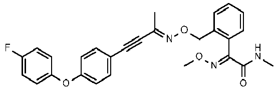 | 2.19 | 474.2 | C |
| 112 | 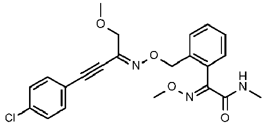 | 2.73 | 428 | E |
| 113 | 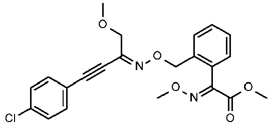 | 2.94 | 429.1 | D |
| 114 | 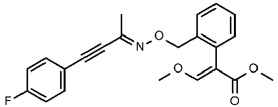 | 2.15 | 382 | A |
| 115 | 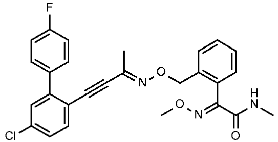 | 2.47 | 492.2 | C |
| 116 | 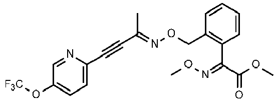 | 1.74 | 450.2 | C |
| 117 | 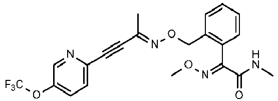 | 3.16 | 449.1 | B |
| 118 | 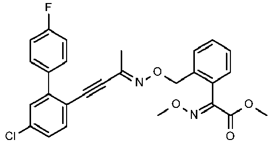 | 2.78 | 493.2 | C |
| 119 | 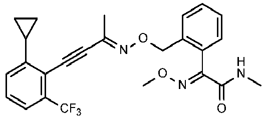 | 2.13 | 472.2 | C |
| 120 | 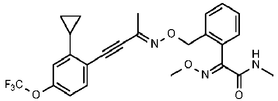 | 3.10 | 488.2 | D |
| 121 | 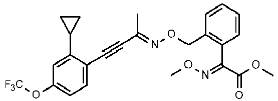 | 3.23 | 489.2 | D |
| 122 | 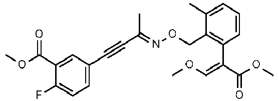 | 2.13 | 454 | A |
| 123 | 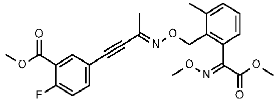 | 2.12 | 455 | A |
| 124 | 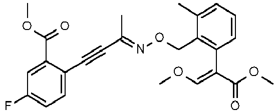 | 2.12 | 454 | A |
| 125 | 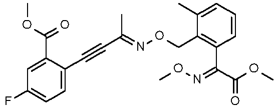 | 2.01 | 455 | A |
| 126 | 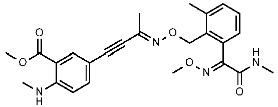 | 1.90 | 464 | A |
| 127 | 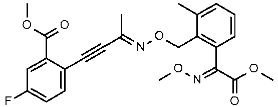 | 2.01 | 455 | A |
| 128 | 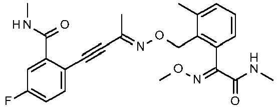 | 1.89 | 453 | A |

### Biological studies

### Green House

The compound was dissolved in a mixture of acetone and/or dimethylsulfoxide and the wetting agent/emulsifier Wettol, which is based on ethoxylated alkylphenoles, in a ratio (volume) solvent-emulsifier of 99 to 1 to give a total volume of 5 ml. Subsequently, water was added to total volume of 100 ml. This stock solution was then diluted with the described solvent-emulsifier-water mixture to the final concentration given in the table below.

### Use example 1. Protective control of soybean rust on soybeans caused by Phakopsora pachyrhizi (PHAKPA P2)

Leaves of potted soybean seedlings were sprayed to run-off with the previously described spray solution, containing the concentration of active ingredient or their mixture as described below. The plants were allowed to air-dry. The trial plants were cultivated for 2 days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 20 to 24 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

### Use example 2. Protective control of soybean rust on soybeans caused by Phakopsora pachyrhizi (PHAKPA P6)

Leaves of potted soybean seedlings were sprayed to run-off with the previously described spray solution, containing the concentration of active ingredient as described below. The plants were allowed to air-dry. The trial plants were cultivated for six days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 23 to 27 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

The results of the abovementioned use examples are given in the following Tables. All test results below are given for the control of phytopathogenic fungi containing the amino acid substitution F129L in the mitochondrial cytochrome b protein conferring resistance to Qo inhibitors.

**Table 1:**

| | | **PHAKPA Disease level (%)** | |
|---|---|---|---|
| **No.** | **Structure** | **P2 at 16 ppm** | **P6 at 16 ppm** |
| 1 | 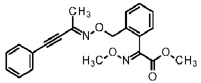 | 38 | 58 |
| 2 | 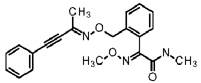 | 97 | 90 |
| 3 | 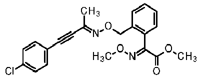 | 70 | 70 |
| 4 | 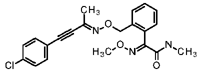 | 3 | 7 |
| 5 | 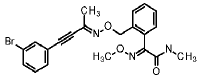 | 1 | 7 |
| 6 | 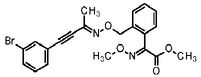 | 25 | 43 |
| 7 | 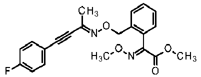 | 30 | 50 |
| 8 | 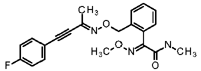 | 0 | 0.7 |
| 9 | 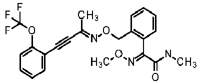 | 0 | 5 |
| 10 | 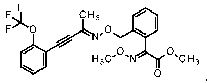 | 70 | 83 |
| 11 | 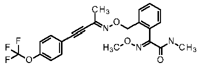 | 53 | 67 |
| 12 | 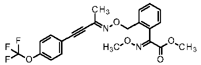 | 93 | 100 |
| 16 | 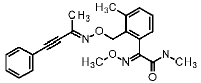 | 0 | 0 |
| 19 | 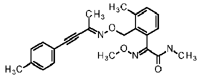 | 0 | 0.6 |
| 20 | 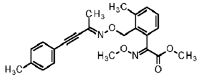 | 2 | 1 |
| 21 | 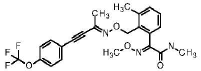 | 25 | 33 |
| 22 | 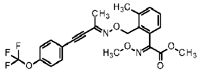 | 93 | 97 |
| 23 | 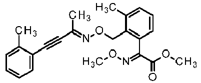 | 0 | 0 |
| 25 | 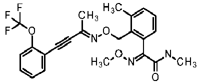 | 0 | 0 |
| 26 | 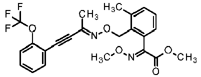 | 2 | 6 |
| 27 | 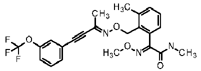 | 0 | 0 |
| 28 | 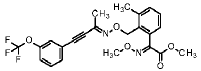 | 0 | 0 |
| 29 | 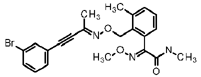 | 1 | 0 |
| 30 | 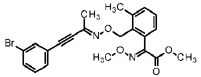 | 0 | 0 |
| 31 | 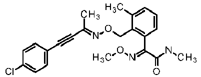 | 0 | 5 |
| 32 | 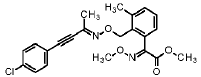 | 12 | 35 |
| 33 | 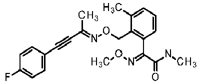 | 0 | 0 |
| 34 | 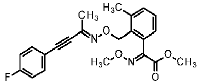 | 0 | 0 |
| 35 | 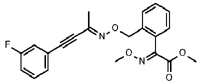 | 32 | 25 |
| 36 | 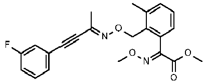 | 4 | 9 |
| 37 | 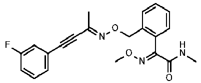 | 6 | 8 |
| 38 | 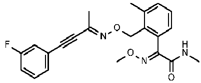 | 0 | 1 |
| 39 | 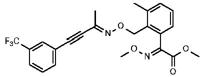 | 2 | 1 |
| 40 | 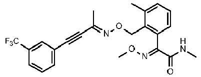 | 0 | 0 |
| 41 | 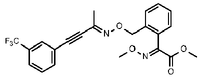 | 50 | 67 |
| 42 | 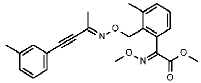 | 3 | 6 |
| 43 | 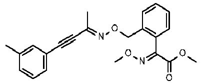 | 40 | 27 |
| 44 | 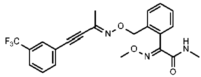 | 1 | 5 |
| 45 | 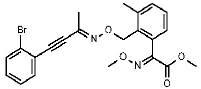 | 25 | 22 |
| 46 | 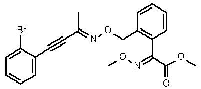 | 47 | 37 |
| 47 | 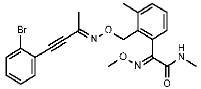 | 0 | 0 |
| 48 | 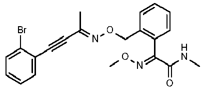 | 0 | 2 |
| 49 | 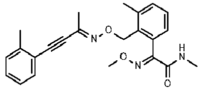 | 0 | 0 |
| 51 | 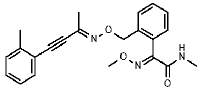 | 0 | 0 |
| 52 | 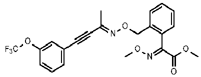 | 13 | 33 |
| 53 | 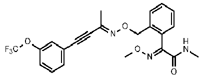 | 0 | 0 |
| 54 | 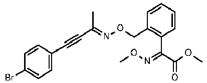 | 22 | 25 |
| 55 | 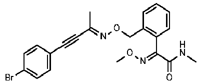 | 23 | 40 |
| 56 | 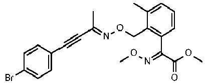 | 18 | 37 |
| 57 | 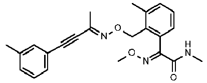 | 0 | 0 |
| 58 | 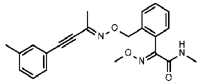 | 2 | 4 |
| 59 | 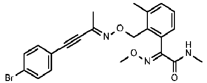 | 13 | 28 |
| 60 | 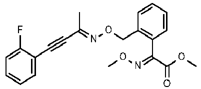 | 40 | 17 |
| 61 | 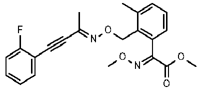 | 8 | 8 |
| 62 | 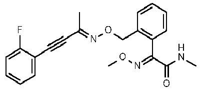 | 2 | 2 |
| 63 | 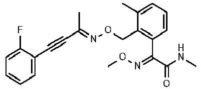 | 0 | 0 |
| 64 | 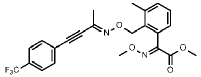 | 43 | 60 |
| 65 | 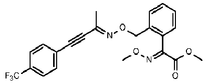 | 80 | 70 |
| 66 | 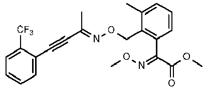 | 17 | 30 |
| 67 | 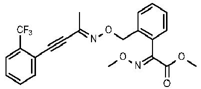 | 50 | 60 |
| 68 | 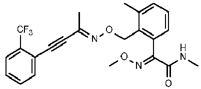 | 0 | 1 |
| 69 | 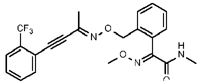 | 1 | 2 |
| 70 | 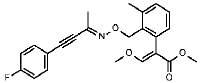 | 0 | 0 |
| 71 | 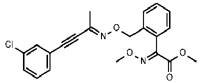 | 20 | 17 |
| 72 | 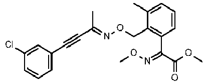 | 7 | 8 |
| 73 | 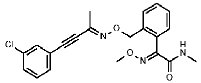 | 1 | 0 |
| 74 | 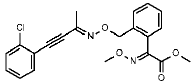 | 42 | 47 |
| 75 | 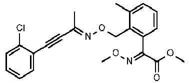 | 22 | 40 |
| 76 | 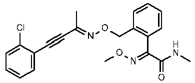 | 3 | 4 |
| 77 | 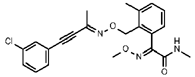 | 0 | 0 |
| 78 | 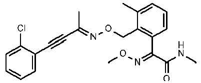 | 0 | 0 |
| 79 | 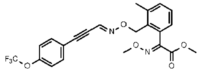 | 67 | 50 |
| 80 | 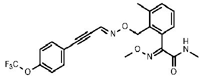 | 43 | 28 |
| 82 | 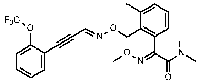 | 4 | 27 |
| 83 | 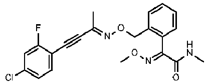 | 17 | 22 |
| 84 | 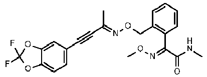 | 77 | 67 |
| 85 | 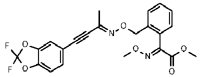 | 100 | 83 |
| 86 | 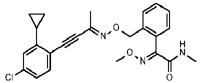 | 5 | 18 |
| 88 | 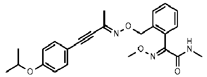 | 77 | 60 |
| 90 | 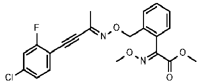 | 30 | 57 |
| 91 | 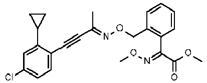 | 53 | 43 |
| 92 | 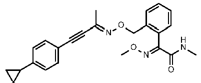 | 60 | 57 |
| 93 | 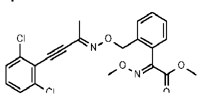 | 87 | 97 |
| 94 | 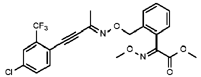 | 53 | 47 |
| 98 | 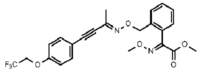 | 73 | 93 |
| 99 | 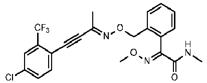 | 0 | 0 |
| 100 | 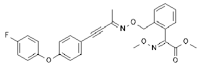 | 77 | 77 |
| 101 | 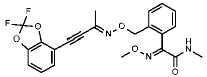 | 4 | 2 |
| 102 | 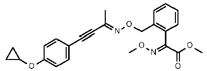 | 80 | 63 |
| 103 | 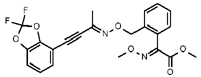 | 28 | 22 |
| 104 | 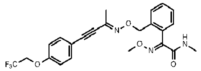 | 53 | 32 |
| 106 | 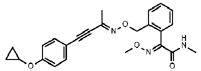 | 23 | 13 |
| 107 | 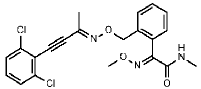 | 27 | 15 |
| 108 | 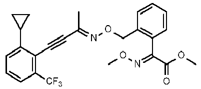 | 90 | 87 |
| 109 | 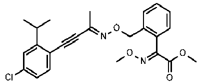 | 11 | 12 |
| 110 | 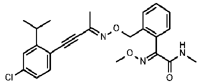 | 0 | 2 |
| 111 | 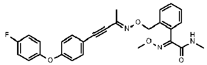 | 33 | 23 |
| 112 | 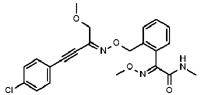 | 38 | 25 |
| 113 | 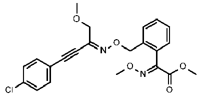 | 87 | 77 |
| 114 | 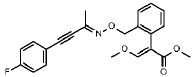 | 0 | 0 |
| 115 | 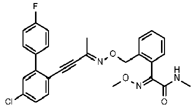 | 2 | 1 |
| 116 | 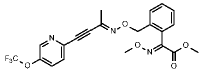 | 80 | 67 |
| 117 | 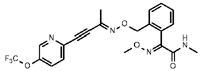 | 77 | 63 |
| 118 | 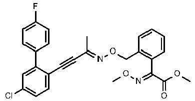 | 3 | 4 |
| 119 | 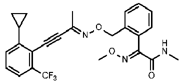 | 77 | 77 |
| 120 | 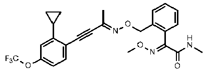 | 15 | 22 |
| 121 | 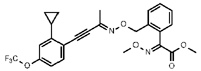 | 87 | 53 |
| 122 | 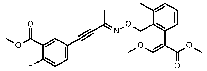 | 73 | 60 |
| 123 | 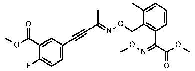 | 43 | 37 |
| 124 | 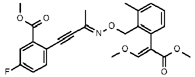 | 14 | 53 |
| 125 | 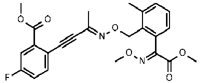 | 17 | 45 |
| 127 | 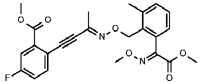 | 53 | 47 |

## Claims

1. Compounds of formula I 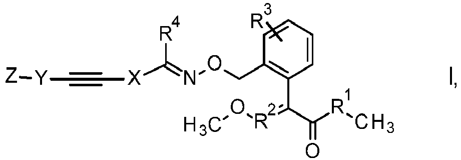 wherein
R¹ is selected from O and NH;
R² is selected from CH and N;
R³ is selected from hydrogen, halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-al kyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
R⁴ is selected from hydrogen, C₁-C₆-alkyl, C₂-C₄-alkenyl, C₂-C₄-alkynyl, C₁-C₆-haloalkyl, C₂-C₄-haloalkenyl, C₂-C₄-haloalkynyl, -(C₁-C₂-alkyl)-O-(C₁-C₂-alkyl), -(C₁-C₂-alkyl)-O-(C₁-C₂-haloalkyl) and -C₁-C₄-alkyl-C₃-C₆-cycloalkyl;
X, Y , independently of each other, are a direct bond or the divalent group -CL¹L²-;
L¹, L², independently of each other, are selected from hydrogen, C₁-C₃-alkyl, C₁-C₃-haloalkyl, C₂-C₃-haloalkenyl, C₂-C₃-haloalkynyl, -(C₁-C₂-alkyl)-O-(C₁-C₂-alkyl), -(C₁-C₂-alkyl)-O-(C₁-C₂-haloalkyl), cyclopropyl and -C₁-C₂-alkyl-cyclopropyl; or
L¹ and L², together with the interjacent carbon atom, form a cyclopropyl; wherein the cyclic moieties of L¹ and L², independently of each other, are unsubstituted or carry 1 or 2 identical or different groups R^{L}:
R^{L} is selected from halogen, CN, NO₂, C₁-C₄-alkyl, C₁-C₄-haloalkyl, -O-C₁-C₄-alkyl, and -O-C₁-C₄-haloalkyl;
Z is selected from C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
and wherein Z is unsubstituted or carries 1, 2, 3 or up to the maximum number of identical or different groups R^{a}:
R^{a} is selected from halogen, CN, NR^{A}R^{B}, C₁-C₄-alkyl, C₂-C₄-alkenyl, C₂-C₄-alkynyl, -O-CᵣC₄-alkyl, -C(=N-O-C₁-C₄-alkyl)-C₁-C₄-alkyl, -C(=O)-C₁₋C₄-alkyl, -C(=O)-O-C₁-C₄-alkyl, -C(=O)-NH-C₁-C₄-alkyl, -O-CH₂-C(= N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl, heterocycloalkenyl and heteroaryl are bound directly or via an oxygen atom or via a C₁-C₂-alkylene linker,
and/or
2 R^{a} substituents bound to neighboring carbon ring atoms, together with the two interjacent carbon ring atoms, form a partially unsaturated or aromatic 5-to 6-membered fused carbo- or heterocycle,
wherein the heterocycle includes beside carbon atoms 1 or 2 heteroatoms independently selected from N, O and S as ring member atoms, provided that such heterocycle cannot contain 2 contiguous atoms selected from O and S;
and wherein the aliphatic and cyclic moieties of R^{a} are unsubstituted or carry 1, 2, 3, 4 or up to the maximum number of identical or different groups R^{b}:
R^{b} is selected from halogen, CN, NO₂, C₁-C₄-alkyl, C₁-C₄-haloalkyl, -O-C₁-C₄-alkyl, and -O-C₁-C₄-haloalkyl;
R^{A}, R^{B} independently of each other, are selected from hydrogen, C₁-C₄-alkyl and C₁-C₄-haloalkyl;
and in form or stereoisomers and tautomers thereof, and the N-oxides and the agriculturally acceptable salts thereof.

2. The compounds according to claim 1, wherein R¹ is selected from O and NH; and R² is selected from CH and N, provided that R² is N in case R¹ is NH.

3. The compounds according to any one of the claims 1 to 2, wherein R² is N.

4. The compounds according to any one of the claims 1 to 3, wherein R³ is selected from hydrogen, CN, halogen, C₁-C₂-alkyl, C₁-C₂-haloalkyl, C₂-C₄-alkenyl, C₃-C₄-cycloalkyl, -O-C₁-C₂-alkyl and -O-C₁-C₂-haloalkyl.

5. The compounds according to claim 4, wherein R³ is selected from hydrogen, halogen, C₁-C₂-alkyl, and C₁-C₂-haloalkyl.

6. The compounds according to any one of the claims 1 to 5, wherein R³ is ortho position to the methyl oxime side chain.

7. The compounds according to any one of the claims 1 to 6, wherein R⁴ is selected from halogen, C₁-C₂-alkyl, C₁-C₂-haloalkyl, C₃-C₄-cycloalkyl, -O-C₁-C₂-alkyl and -O-C₁-C₂-haloalkyl.

8. The compounds according to any one of the claims 1 to 7, wherein X is a direct bond.

9. The compounds according to any one of the claims 1 to 8, wherein Y is a direct bond.

10. The compounds according to any one of the claims 1 to 9, wherein Z is selected from C₃-C₄-cycloalkyl, phenyl and 5- or 6-membered heteroaryl, wherein said heteroaryl besides carbon atoms contain 1, 2 or 3 heteroatoms selected from N, O and S provided that such heterocycle cannot contain 2 contiguous atoms selected from O and S, wherein said phenyl, and wherein Z is unsubstituted or carries 1, 2 or 3 identical or different groups R^{a} selected from halogen, CN, C₁-C₂-alkyl, C₁-C₂-haloalkyl, -O-C₁-C₂-alkyl, -O-C₁-C₂-haloalkyl, C₃-C₄-cycloalkyl and C₃-C₄-halocycloalkyl.

11. The compounds according to claim 10, wherein Z is phenyl, and wherein Z is unsubstituted or carries 1, 2 or 3 identical or different groups R^{a} selected from halogen, CN, C₁-C₂-alkyl, C₁-C₂-haloalkyl, -O-C₁-C₂-alkyl, -O-C₁-C₂-haloalkyl, C₃-C₄-cycloalkyl and C₃-C₄-halocycloalkyl.

12. Agrochemical compositions comprising an auxiliary and at least one compound of formula I as defined in any of claims 1 to 11 or in the form of a stereoisomer and tautomer thereof or an agriculturally acceptable salt or N-oxide thereof.

13. Use of the compounds as defined in any of the claims 1 to 11 or a composition as defined in claim 12 for combating phytopathogenic fungi.

14. A method for combating phytopathogenic fungi comprising:
treating curatively and/or preventively the plants or the plant propagation material of said plants that are at risk of being diseased from the said phytopathogenic fungi, and/or
applying to the said phytopathogenic fungi, at least one compound of formula I as defined in any of the claims 1 to 11 or an agrochemical composition as defined in claim 12.

15. The use according to claim 13 or the method according to claim 14, wherein the phytopathogenic fungi contain an amino acid substitution F129L in the mitochondrial cytochrome b protein conferring resistance to Qo inhibitors.

## Patentansprüche

1. Verbindungen der Formel I 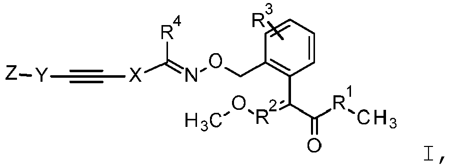 wobei
R¹ aus O und NH ausgewählt ist,
R² aus CH und N ausgewählt ist,
R³ aus Wasserstoff, Halogen, CN, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₃-C₆-Cycloalkyl,-O-C₁-C₄-Alkyl, -O-C₁-C₄-Halogenalkyl, -O-C₃-C₆-Cycloalkyl, -C₁-C₂-Alkyl-C₃-C₆-cycloalkyl, Phenyl, 3- bis 6-gliedrigem Heterocycloalkyl und 5- oder 6-gliedrigem Heteroaryl ausgewählt ist, wobei Heterocycloalkyl und Heteroaryl neben Kohlenstoffatomen 1, 2 oder 3 aus N, O und S ausgewählte Heteroatome enthalten, mit der Maßgabe, dass ein solcher Heterocyclus nicht 2 benachbarte Atome ausgewählt aus O und S enthalten kann,
wobei Phenyl, Heterocycloalkyl und Heteroaryl direkt oder über ein Sauerstoffatom oder über einen C₁-C₂-Alkylen-Linker gebunden sind und wobei Phenyl und Heteroaryl unsubstituiert oder durch 1, 2 oder 3 gleiche oder verschiedene Substituenten ausgewählt aus Halogen, CN, NH₂, NO₂, C₁-C₄-Alkyl, C₁-C₄-Halogenalkyl, -O-C₁-C₄-Alkyl und -O-C₁-C₄-Halogenalkyl substituiert sind,
R⁴ aus Wasserstoff, C₁-C₆-Alkyl, C₂-C₄-Alkenyl, C₂-C₄-Alkinyl, C₁-C₆-Halogenalkyl, C₂-C₄-Halogenalkenyl, C₂-C₄-Halogenalkinyl, - (C₁-C₂-Alkyl)-O-(C₁-C₂-alkyl) , -(C₁-C₂-Alkyl) -O-(C₁-C₂-halogenalkyl) und -C₁-C₄-Alkyl-C₃-C₆-cycloalkyl ausgewählt ist,
X, Y unabhängig voneinander für eine direkte Bindung oder die zweiwertige Gruppe -CL¹L²- stehen,
L¹, L² unabhängig voneinander aus Wasserstoff, C₁-C₃-Alkyl, C₁-C₃-Halogenalkyl, C₂-C₃-Halogenalkenyl, C₂-C₃-Halogenalkinyl, -(C₁-C₂-Alkyl)-O-(C₁-C₂-alkyl), -(C₁-C₂-Alkyl)-O-(C₁-C₂-halogenalkyl), Cyclopropyl und -C₁-C₂-Alkyl-cyclopropyl ausgewählt sind oder
L¹ und L² zusammen mit dem dazwischenliegenden Kohlenstoffatom Cyclopropyl bilden,
wobei die cyclischen Gruppierungen von L¹ und L² unabhängig voneinander unsubstituiert sind oder 1 oder 2 gleiche oder verschiedene R^{L}-Gruppen tragen:
R^{L} aus Halogen, CN, NO₂, C₁-C₄-Alkyl, C₁-C₄-Halogenalkyl, -O-C₁-C₄-Alkylund -O-C₁-C₄-Halogenalkyl ausgewählt ist,
Z aus C₃-C₆-Cycloalkyl, Phenyl, 3- bis 6-gliedrigem Heterocycloalkyl, 3- bis 6-gliedrigem Heterocycloalkenyl und 5- oder 6-gliedrigem Heteroaryl ausgewählt ist,
wobei Heterocycloalkyl, Heterocycloalkenyl und Heteroaryl neben Kohlenstoffatomen 1, 2 oder 3 aus N, O und S ausgewählte Heteroatome enthalten, mit der Maßgabe, dass ein solcher Heterocyclus nicht 2 benachbarte Atome ausgewählt aus O und S enthalten kann,
und wobei Z unsubstituiert ist oder 1, 2, 3 oder bis zur maximalen Anzahl gleiche oder verschiedene R^{a}-Gruppen trägt:
R^{a} aus Halogen, CN, NR^{A}R^{B}, C₁-C₄-Alkyl, C₂-C₄-Alkenyl, C₂-C₄-Alkinyl, -O-C₁-C₄-Alkyl, -C(=N-O-C₁-C₄-Alkyl)-C₁-C₄-alkyl, -C(=O)-C₁-C₄-Alkyl, -C(=O)-O-C₂-C₄-Alkyl, -C(=O)-NH-C₁-C₄-Alkyl, -O-CH₂-C(=N-O-C₁-C₄-Alkyl)-C₁-C₄-alkyl, C₃-C₆-Cycloalkyl, C₃-C₆-Cycloalkenyl, -C₁-C₂-Alkyl-C₃-C₆-cycloalkyl, -O-C₃-C₆-Cycloalkyl, Phenyl, 3- bis 6-gliedrigem Heterocycloalkyl, 3- bis 6-gliedrigem Heterocycloalkenyl und 5- oder 6-gliedrigem Heteroaryl ausgewählt ist,
wobei Heterocycloalkyl, Heterocycloalkenyl und Heteroaryl neben Kohlenstoffatomen 1, 2 oder 3 aus N, O und S ausgewählte Heteroatome enthalten, mit der Maßgabe, dass ein solcher Heterocyclus nicht 2 benachbarte Atome ausgewählt aus O und S enthalten kann,
wobei Phenyl, Heterocycloalkyl, Heterocycloalkenyl und Heteroaryl direkt oder über ein Sauerstoffatom oder über einen C₁-C₂-Alkylen-Linker gebunden sind,
und/oder
2 an benachbarte Kohlenstoffringatome gebundene R^{a}-Substituenten zusammen mit den beiden dazwischenliegenden Kohlenstoffringatomen einen teilweise ungesättigten oder aromatischen 5- bis 6-gliedrigen kondensierten Carbo- oder Heterocyclus bilden,
wobei der Heterocyclus neben Kohlenstoffatomen 1 oder 2 unabhängig voneinander aus N, O und S ausgewählte Heteroatome enthält, mit der Maßgabe, dass ein solcher Heterocyclus nicht 2 benachbarte Atome ausgewählt aus O und S enthalten kann,
und wobei die aliphatischen und cyclischen Gruppierungen von R^{a} unsubstituiert sind oder 1, 2, 3, 4 oder bis zur maximalen Anzahl gleiche oder verschiedene R^{b}-Gruppen tragen:
R^{b} aus Halogen, CN, NO₂, C₁-C₄-Alkyl, C₁-C₄-Halogenalkyl, -O-C₁-C₄-Alkylund -O-C₁-C₄-Halogenalkyl ausgewählt ist,
R^{A}, R^{B} unabhängig voneinander aus Wasserstoff, C₁-C₄-Alkyl und C₁-C₄-Halogenalkyl ausgewählt sind,
und in Form von Stereoisomeren und Tautomeren davon, und die N-Oxide und die landwirtschaftlich unbedenklichen Salze davon.

2. Verbindungen nach Anspruch 1, wobei R¹ aus O und NH ausgewählt ist und R² aus CH und N ausgewählt ist, mit der Maßgabe, dass R² für N steht, wenn R¹ für NH steht.

3. Verbindungen nach einem der Ansprüche 1 bis 2, wobei R² für N steht.

4. Verbindungen nach einem der Ansprüche 1 bis 3, wobei R³ aus Wasserstoff, CN, Halogen, C₁-C₂-Alkyl, C₁-C₂-Halogenalkyl, C₂-C₄-Alkenyl, C₃-C₄-Cycloalkyl, -O-C₁-C₂-Alkyl und -O-C₁-C₂-Halogenalkyl ausgewählt ist.

5. Verbindungen nach Anspruch 4, wobei R³ aus Wasserstoff, Halogen, C₁-C₂-Alkyl und C₁-C₂-Halogenalkyl ausgewählt ist.

6. Verbindungen nach einem der Ansprüche 1 bis 5, wobei R³ in der ortho-Position zur Methyloxim-Seitenkette steht.

7. Verbindungen nach einem der Ansprüche 1 bis 6, wobei R⁴ aus Halogen, C₁-C₂-Alkyl, C₁-C₂-Halogenalkyl, C₃-C₄-Cycloalkyl, -O-C₁-C₂-Alkyl und -O-C₁-C₂-Halogenalkyl ausgewählt ist.

8. Verbindungen nach einem der Ansprüche 1 bis 7, wobei X für eine direkte Bindung steht.

9. Verbindungen nach einem der Ansprüche 1 bis 8, wobei Y für eine direkte Bindung steht.

10. Verbindungen nach einem der Ansprüche 1 bis 9, wobei Z aus C₃-C₄-Cycloalkyl, Phenyl und 5- oder 6-gliedrigem Heteroaryl ausgewählt ist, wobei Heteroaryl neben Kohlenstoffatomen 1, 2 oder 3 aus N, O und S ausgewählte Heteroatome enthält, mit der Maßgabe, dass ein solcher Heterocyclus nicht 2 benachbarte Atome ausgewählt aus O und S enthalten kann, wobei Phenyl und wobei Z unsubstituiert ist oder 1, 2 oder 3 gleiche oder verschiedene R^{a}-Gruppen ausgewählt aus Halogen, CN, C₁-C₂-Alkyl, C₁-C₂-Halogenalkyl, -O-C₁-C₂-Alkyl, -O-C₁-C₂-Halogenalkyl, C₃-C₄-Cycloalkyl und C₃-C₄-Halogencycloalkyl trägt.

11. Verbindungen nach Anspruch 10, wobei Z für Phenyl steht und wobei Z unsubstituiert ist oder 1, 2 oder 3 gleiche oder verschiedene R^{a}-Gruppen ausgewählt aus Halogen, CN, C₁-C₂-Alkyl, C₁-C₂-Halogenalkyl, -O-C₁-C₂-Alkyl, -O-C₁-C₂-Halogenalkyl, C₃-C₄-Cycloalkyl und C₃-C₄-Halogencycloalkyl trägt.

12. Agrochemische Zusammensetzungen, umfassend einen Hilfsstoff und mindestens eine wie in einem der Ansprüche 1 bis 11 definierte Verbindung der Formel I oder in Form eines Stereoisomers und Tautomers davon oder ein landwirtschaftliche unbedenkliches Salz oder N-Oxid davon.

13. Verwendung der wie in einem der Ansprüche 1 bis 11 definierten Verbindungen oder einer wie in Anspruch 12 definierten Zusammensetzung zur Bekämpfung phytopathogener Pilze.

14. Verfahren zur Bekämpfung phytopathogener Pilze, bei dem man:
die Pflanzen oder das Pflanzenfortpflanzungsmaterial der Pflanzen, bei denen ein Risiko einer Erkrankung durch die phytopathogenen Pilze besteht, kurativ und/oder präventiv behandelt, und/oder auf die phytopathogenen Pilze mindestens eine wie in einem der Ansprüche 1 bis 11 definierte Verbindung der Formel I oder eine wie in Anspruch 12 definierte Zusammensetzung aufbringt.

15. Verwendung nach Anspruch 13 oder Verfahren nach Anspruch 14, wobei die phytopathogenen Pilze eine Aminosäuresubstitution F129L im mitochondrialen Cytochrom-b-Protein enthalten, die Resistenz gegenüber Qo-Inhibitoren verleiht.

## Revendications

1. Composés de formule I 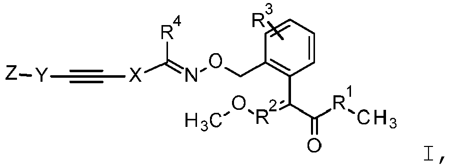
R¹ étant choisi parmi O et NH ;
R² étant choisi parmi CH et N ;
R³ étant choisi parmi hydrogène, halogène, CN, C₁-C₄-alkyle, C₂-C₄-alcényle, C₂-C₄-alcynyle, C₁-C₄-halogénoalkyle, C₂-C₄-halogénoalcényle, C₂-C₄-halogénoalcynyle, C₃-C₆-cycloalkyle, -O-C₁-C₄-alkyle, -O-C₁-C₄-halogénoalkyle, -O-C₃-C₆-cycloalkyle, -C₁-C₂-alkylC₃-C₆-cyclo-alkyle, phényle, hétérocycloalkyle à 3 à 6 chaînons et hétéroaryle à 5 ou 6 chaînons, lesdits hétérocycloalkyle et hétéroaryle, outre les atomes de carbone contenant 1, 2 ou 3 hétéroatomes choisis parmi N, O et S à condition qu'un tel hétérocycle ne puisse pas contenir 2 atomes contigus choisis parmi O et S,
lesdits phényle, hétérocycloalkyle et hétéroaryle étant liés directement ou via un atome d'oxygène ou via un lieur C₁-C₂-alkylène, et lesdits phényle et hétéroaryle étant non substitués ou substitués par 1, 2 ou 3 substituants identiques ou différents choisis parmi halogène, CN, NH₂, NO₂, C₁-C₄-alkyle, C₁-C₄-halogénoalkyle, -O-C₁-C₄-alkyle et -O-C₁-C₄-halogénoalkyle ;
R⁴ étant choisi parmi hydrogène, C₁-C₆-alkyle, C₂-C₄-alcényle, C₂-C₄-alcynyle, C₁-C₆-halogénoalkyle, C₂-C₄-halogénoalcényle, C₂-C₄-halogénoalcynyle, -(C₁-C₂-alkyl)-O-(C₁-C₂-alkyle), -(C₁-C₂-alkyl)-O-(C₁-C₂-halogénoalkyle) et -C₁-C₄-alkyl-C₃-C₆-cycloalkyle ;
X, Y, indépendamment l'un de l'autre, étant une liaison directe ou le groupe divalent -CL¹L²- ;
L¹, L², indépendamment l'un de l'autre, étant choisis parmi hydrogène, C₁-C₃-alkyle, C₁-C₃-halogéno-alkyle, C₂-C₃-halogénoalcényle, C₂-C₃-halogénoalcynyle, - (C₁-C₂-alkyl)-O-(C₁-C₂-alkyle), -(C₁-C₂-alkyl)-O-(C₁-C₂-halogénoalkyle), cyclopropyle et -C₁-C₂-alkyl-cyclopropyle ; ou
L¹ et L², conjointement avec l'atome de carbone interjacent, formant un cyclopropyle ;
les groupements cycliques de L¹ et L², indépendamment l'un de l'autre, étant non substitués ou portant 1 ou 2 groupes R^{L} identiques ou différents :
R^{L} étant choisi parmi halogène, CN, NO₂, C₁-C₄-alkyle, C₁-C₄-halogénoalkyle, -O-C₁-C₄-alkyle, et -O-C₁-C₄-halogénoalkyle ;
Z étant choisi parmi C₃-C₆-cycloalkyle, phényle, hétérocycloalkyle à 3 à 6 chaînons, hétérocycloalcényle à 3 à 6 chaînons et hétéroaryle à 5 ou 6 chaînons,
lesdits hétérocycloalkyle, hétérocycloalcényle et hétéroaryle, outre les atomes de carbone contenant 1, 2 ou 3 hétéroatomes choisis parmi N, O et S à condition qu'un tel hétérocycle ne puisse pas contenir 2 atomes contigus choisis parmi O et S,
et Z étant non substitué ou portant 1, 2, 3 ou jusqu'au nombre maximal de groupes R^{a} identiques ou différents :
R^{a} étant choisi parmi halogène, CN, NR^{A}R^{B}, C₁-C₄-alkyle, C₂-C₄-alcényle, C₂-C₄-al-cynyle, -O-C₁-C₄-alkyle, -C(=N-OC₁-C₄-alkyl) -C₁-C₄-alkyle, -C(=O)-C₁-C₄-alkyle, -C(=O)-OC₁-C₄-alkyle, -C(=O)-NH-C₁-C₄-alkyle,
-O-CH₂-C(=N-O-C₁-C₄-alkyl)-C₁-C₄-alkyle, C₃-C₆-cycloalkyle, C₃-C₆-cycloalcényle, -C₁-C₂-alkyl-C₃-C₆-cycloalkyle, -O-C₃-C₆-cycloalkyle, phényle, hétérocycloalkyle à 3 à 6 chaînons, hétérocycloalcényle à 3 à 6 chaînons et hétéroaryle à 5 ou 6 chaînons, lesdits hétérocycloalkyle, hétérocycloalcényle et hétéroaryle, outre les atomes de carbone contenant 1, 2 ou 3 hétéroatomes choisis parmi N, O et S à condition qu'un tel hétérocycle ne puisse pas contenir 2 atomes contigus choisis parmi O et S,
lesdits phényle, hétérocycloalkyle, hétérocycloalcényle et hétéroaryle étant liés directement ou via un atome d'oxygène ou via un lieur C₁-C₂-alkylène,
et/ou
2 substituants R^{a} liés à des atomes de cycle de carbone adjacents, conjointement avec les deux atomes de cycle de carbone interjacents, formant un carbocycle ou hétérocycle condensé à 5 ou 6 chaînons partiellement insaturé ou aromatique,
l'hétérocycle comprenant outre des atomes de carbone 1 ou 2 hétéroatomes indépendamment choisi(s) parmi N, O et S en tant qu'atomes d'éléments de cycle, à condition qu'un tel hétérocycle ne puisse pas contenir 2 atomes contigus choisis parmi O et S ;
et les groupements aliphatiques et cycliques de R^{a} étant non substitués ou portant 1, 2, 3, 4 ou jusqu'au nombre maximal de groupes R^{b} identiques ou différents :
R^{b} étant choisi parmi halogène, CN, NO₂, C₁-C₄-alkyle, C₁-C₄-halogénoalkyle, -O-C₁-C₄-alkyle, et -O-C₁-C₄-halogénoalkyle ;
R^{A}, R^{B} indépendamment l'un de l'autre, étant choisis parmi hydrogène, C₁-C₄-alkyle et C₁-C₄-halogénoalkyle ;
et sous forme de stéréoisomères et de formes tautomère correspondant(e)s, et les N-oxydes et les sels acceptables sur le plan agricole correspondants.

2. Composés selon la revendication 1, R¹ étant choisi parmi O et NH ; et R² étant choisi parmi CH et N, à condition que R² soit N dans le cas où R¹ est NH.

3. Composés selon l'une quelconque des revendications 1 à 2, R² étant N.

4. Composés selon l'une quelconque des revendications 1 à 3, R³ étant choisi parmi hydrogène, CN, halogène, C₁-C₂-alkyle, C₁-C₂-halogénoalkyle, C₂-C₄-alcényle, C₃-C₄-cycloalkyle, -O-C₁-C₂-alkyle et -O-C₁-C₂-halogénoalkyle.

5. Composés selon la revendication 4, R³ étant choisi parmi hydrogène, halogène, C₁-C₂-alkyle, et C₁-C₂-halogénoalkyle.

6. Composés selon l'une quelconque des revendications 1 à 5, R³ étant en position ortho par rapport à la chaîne latérale méthyloxime.

7. Composés selon l'une quelconque des revendications 1 à 6, R⁴ étant choisi parmi halogène, C₁-C₂-alkyle, C₁-C₂-halogénoalkyle, C₃-C₄-cycloalkyle, -O-C₁-C₂-alkyle et - O-C₁-C₂-halogénoalkyle.

8. Composés selon l'une quelconque des revendications 1 à 7, X étant une liaison directe.

9. Composés selon l'une quelconque des revendications 1 à 8, Y étant une liaison directe.

10. Composés selon l'une quelconque des revendications 1 à 9, Z étant choisi parmi C₃-C₄-cycloalkyle, phényle et hétéroaryle à 5 ou 6 chaînons, lesdits hétéroaryle, outre les atomes de carbone contenant 1, 2 ou 3 hétéroatomes choisis parmi N, O et S à condition qu'un tel hétérocycle ne puisse pas contenir 2 atomes contigus choisis parmi O et S, lesdits phényle, et Z étant non substitué ou portant 1, 2 ou 3 groupes R^{a} identiques ou différents choisis parmi halogène, CN, C₁-C₂-alkyle, C₁-C₂-halogénoalkyle, - O-C₁-C₂-alkyle, -O-C₁-C₂-halogéno-alkyle, C₃-C₄-cycloalkyle et C₃-C₄-halogénocycloalkyle.

11. Composés selon la revendication 10, Z étant phényle, et Z étant non substitué ou portant 1, 2 ou 3 groupes R^{a} identiques ou différents choisis parmi halogène, CN, C₁-C₂-alkyle, C₁-C₂-halogénoalkyle, -O-C₁-C₂-alkyle, -O-C₁-C₂-halogénoalkyle, C₃-C₄-cycloalkyle et C₃-C₄-halogénocycloalkyle.

12. Compositions agrochimiques comprenant un auxiliaire et au moins un composé de formule I tel que défini dans l'une quelconque des revendications 1 à 11 ou sous la forme d'un stéréoisomère et d'une forme tautomère correspondant (e) ou d'un sel acceptable sur le plan agricole ou d'un N-oxyde correspondant.

13. Utilisation des composés tels que définis dans l'une quelconque des revendications 1 à 11 ou d'une composition telle que définie dans la revendication 12 pour la lutte contre des champignons phytopathogènes.

14. Procédé de lutte contre des champignons phytopathogènes comprenant :
le traitement de manière curative et/ou préventive des végétaux ou du matériel de propagation végétale desdits végétaux qui sont à risque d'être malades par lesdits champignons phytopathogènes, et/ou l'application sur lesdits champignons phytopathogènes, d'au moins un composé de formule I tel que défini dans l'une quelconque des revendications 1 à 11 ou d'une composition agrochimique telle que définie dans la revendication 12.

15. Utilisation selon la revendication 13 ou procédé selon la revendication 14, dans laquelle/lequel les champignons phytopathogènes contiennent une substitution d'acide aminé F129L dans la protéine mitochondriale du cytochrome b conférant une résistance aux inhibiteurs de Qo.
